# EUROPEAN PATENT SPECIFICATION

(11) **EP 1 217 888 B1**
(45) Date of publication and mention of the grant of the patent: **01.09.2004**
(21) Application number: 00944693.1
(22) Date of filing: 16.06.2000
(51) Int. Cl.: A01L 9/00

(54) **HEATED VOLATILE DISPENSER**
GEHEIZTE VORRICHTUNG ZUR ABGABE VON FLÜCHTIGEN STOFFEN
DIFFUSEUR DE PRODUIT VOLATILE CHAUFFES

(30) Priority: 17.06.1999 US 335370; 25.05.2000 US 579409
(43) Date of publication of application: 03.07.2002
(73) Proprietor: S.C. JOHNSON & SON, INC., Racine, Wisconsin 53403-5011 (US)
(72) Inventor: SOLLER, Douglas, A., Racine, WI 53406 (US); FRYAN, Michael, C., Racine, WI 53406 (US); LEONARD, Stephen, B., Franksville, WI 53126 (US); DEMAREST, Scott, W., Racine, WI 53108 (US); MINEAU, Steven, B., Waterford, WI 53185 (US); FURNER, Paul, E., Racine, WI 53406 (US); SHANKLIN, Donald, J., Fullerton, CA 92831 (US)
(74) Representative: Ruschke, Hans Edvard, Dipl.-Ing.
(86) International application number: PCT/US2000/016585
(87) International publication number: WO 2000/078135

(56) References cited:
- WO-A-92/06594
- DE-U- 29 720 802
- FR-A- 2 537 394
- US-A- 2 942 090
- US-A- 4 750 471
- US-A- 6 033 212

## Description

The present invention relates to dispensers for volatiles such as scents, insect control active ingredients, and the like. In particular, it relates to such dispensers that use a fuel burner.

There are a variety of known dispensers for volatiles that employ heat from a flame or from catalyzed combustion to dispense volatiles from volatile-impregnated substrates. Citronella candles mix the volatile into the fuel itself. However, this leaves the candle flame exposed.

U.S. patent 692,075 shows the use of heat from the flame of a conventional oil lamp to dispense volatile ingredients held exposed to the ambient air on a mesh mounted on a lamp chimney, above the lamp's flame. The disclosure of this patent and of all other publications referred to herein are incorporated by reference as if fully set forth herein. The volatile material being heated by this device is positioned above the lamp chimney and thus is directly exposed to ambient air currents, which can cause uneven heating and cooling of the volatile material. The exposed location of the material being heated also allows it to be touched or disturbed by a passing child or animal. Furthermore, it is immediately visible to a user so that charred material can present an unsavory sight.

U.S. patent 143,583 discloses a fumigator in which an alcohol lamp is placed at the bottom of a metal chimney. A cup to hold an otherwise uncontained liquid fumigant is suspended within the chimney at its top, and a perforated lid closes the chimney. The lamp heats the liquid fumigant, and vapor escapes through the perforations of the lid. Handling the uncontained liquid fumigant and gaining access to and refilling the cup can be inconvenient and risk spillage.

Petzwinkler, South African patent abstract 94/5537, discloses an oil lamp equipped with a metal mosquito mat holder that is positioned beside, as opposed to over, the lamp's flame. Heat radiating from the flame heats a metal holder from that side of the holder which restrict access into the slot. To enter, a tray must present a compementary cross-sectional profile to the slot.

Document US-A-4,750,471 concerns a volatile dispenser in which the volatile carrier is mounted essentially in the ceiling of a heating chamber. Gasses from a catalytic heater within the heating chamber pass the underside of the volatile carrier and thereafter pass through openings in the ceiling. There is no flow of hot gasses over the upper surface of the volatile carrier. An arrangement such as this suffers from the disadvantage that the upper surface of the volatile carrier is exposed to outside influences, for example interference by children, or by external breezes. Also, it is susceptible to preferential volatilization from the underside of the mat where the hot gasses pass, which can become depleted before the outward-facing surface looses its active ingredient.

Document FR-A-2 537 394 shows a volatile dispenser in which the volatile carrier is heated from below by the heat from a gas lamp. The outer surface of the volatile carrier is completely exposed to the environment.

Document DE 297 20 802 -U1 discloses a lamp containing a candle which has in an intermediate position up the chimney an annular shelf on which rests a volatile carrier. Evaporation from the volatile carrier is activated by heating effect acting on the underside of the shelf. is presented toward the flame. A conventional mosquito mat is then held vertically on the opposite side of the holder, away from the flame. By this means, the mat is shielded from direct exposure to the flame or its gases, albeit it is heated to drive off the volatiles contained in the mat.

The Petzwinkler dispenser provides a visible flame. However, this dispenser has a mosquito mat holder that holds a mat beside the flame, in open view of a user, detracting from the pleasing visual effect of the flame itself. Also, one must remove the dispenser's chimney to gain access to a spent mat to replace it.

U.S. patents 5,700,430 and 3,778,924 each employ butane as a fuel for a flame or a catalytic burner, using a replaceable fuel tank. In 5,700,430 a mosquito mat is laid on top of a metal plate. Heat is conducted from the location of a flame to the metal plate by means of intervening, heat-conductive parts. In a subsequent version of the device that otherwise closely corresponded to the embodiment shown, a butane flame was enclosed within a metal, rectangular, open-ended box. The box was heated by the flame, and the flame's gases exited an open end of the box to be vented from the device. A mosquito mat was positioned on top of the box to receive heat conducted through the metal box from the flame. The butane flame, heat-conductive parts, and mosquito mat were all held within a protective heat box.

U.S. patent 5,700,430 thus relies on indirect heating. The volatiles from the hottest places on the mat are released fastest. Consequently, the mat's volatiles are discharged unevenly, with the possibility that volatiles at locations remote from the hottest places may never be discharged before the mat's overall release rate becomes so low as to require replacement of the mat.

In U.S. patent 3,778,924 a mosquito mat is held exposed to the ambient air on a metal sole plate over a catalytic burner fueled by butane drawn from a replaceable, pressurized tank. However, the mat is not enclosed in a heating chamber.

Other patents disclose assemblies that rely on an electrical heater (as distinguished from a fuel burning heater) to heat the volatile carrier. See e.g. U.S. patents 2,513,919, 2,942,090, 4,849,181 and 5,111,477. This restricts the portability of the device (it cannot easily be used at camping or picnic sites which do not have electrical power).

U.S. patent 5,722,199 discloses a flea trap (without a volatile heater) having a removable tray that slides into a slot in the flea trap. The slot has keying structures that

There are also a number of other known insect repellent/killing devices which provide a heat source under a platform designed to support a pad that has been impregnated with the insect control active ingredient. Some use a liquid fuel such as alcohol that is burned in an open flame, or directed to a catalyst mesh where it combusts.

In some cases the platform is an open grid. In others it is a flat metal plate heated from beneath. Some of these systems also provide a separate grid structure which snaps or swings over the carrier for restricting access to the heated mat during operation. These systems typically do not provide a light source through transparent sides of a heating chamber (e.g. they are designed purely for insect control).

It can therefore be seen that there is a need for an improved heated volatile dispenser.

### BRIEF SUMMARY OF THE INVENTION

The invention provides a heated volatile dispenser for dispensing volatile ingredients from a volatile carrier. "Volatile ingredients" include (without limitation) perfumes and other air quality modifying materials, as well as insect control ingredients. "Insect" includes arachnids and other similar, small animals commonly controlled in conjunction with insects. "Insect control ingredients" are defined as including (without limitation) insecticides, repellents, and other development or behavior modifying materials. One highly preferred insect control agent is d-cis/trans allethrin.

A "volatile carrier" is a material or structure for holding a volatile ingredient for dispensing. "Mats" are one common type of volatile carrier often used with insect control ingredients and are defined as including (without limitation) woven, felted, or otherwise formed fibrous or cellulose materials; as well as molded, extruded, cast, or otherwise formed polymeric, ceramic, and clay materials, together with other convenient materials loaded with volatile ingredients, whether by impregnation, printing, or otherwise. Volatile carriers can also be metal or plastic cups holding a volatilizable gel; cups holding a gel, powder, or liquid retained in the cup by a volatile-permeable membrane; or any other convenient means for holding a material to be volatilized by the application of heat. However, uncontained liquids or powders, together with liquids or powders held in open cups or similar containers, are excluded from the term "volatile carrier," as used herein.

In one form, the heated volatile dispenser of the invention has an enclosed heating chamber having chamber walls. The heating chamber preferably also has a ceiling, although a heating chamber will be understood as being "enclosed" if it has walls, either an open top or a ceiling, and either a closed or an open bottom. If the heating chamber has a ceiling, the heating chamber also has exit vents in the ceiling or chamber walls or both that communicate between the interior of the heating chamber and the outside air. The exit vents are holes, slots, or other openings that function as vents. Particularly preferred are permanently enclosed structures with ceilings.

The dispenser also has a heat source that preferably is a fuel burner. The fuel burner can be a candle, a burner using a solidified combustible liquid such as conventional gelled alcohol, a burnable solid, a pressurized gas burner, a wick that is fueled with a combustible liquid, a catalytic heater burning a gas or liquid fuel, or any other convenient means for combusting a fuel.

The heated volatile dispenser is equipped to hold a volatile carrier contained within the heating chamber. It is possible to design a volatile carrier that requires no separate, specific structure in the dispenser to hold it within the heating chamber--for example, a volatile carrier equipped with side hooks or arms that hook over the tops of the heating chamber walls, allowing the rest of the volatile carrier to hang downwardly within the heating chamber. Such side hooks, together with the surface that supports them, would constitute a carrier holder. However, it is preferred that the heated volatile dispenser include an additional structure that serves as a carrier holder that is positioned to receive and hold a volatile carrier at a location above the fuel burner and contained within the heating chamber. An air-flow path is provided to guide hot gases, rising from the fuel burner by convection, past the location where a volatile carrier can be held, whether or not in a separate carrier holder, to heat the volatile carrier. The air-flow path is preferably defined, at least in part, by internal surfaces of the heating chamber walls. Heating is accomplished by the direct exposure of the volatile carrier to gases heated by the fuel burner. Preferably, the hot gases include combustion products from the fuel burner. The air-flow path then directs the hot gases through the open top of an open-topped heating chamber or through the exit vents, if a ceiling is present, to escape from the dispenser. As the volatile carrier is heated by the gases, volatile material is released and is carried out of the dispenser with the escaping hot gases.

As indicated above, the term "carrier holder" should be understood very broadly as including any structure that provides for positioning a volatile carrier within the air-flow path, within the heating chamber. In some embodiments, very little separate structure is actually required. For example, in one form, the carrier holder can be a slot in the heating chamber wall through which a volatile carrier is inserted, with the slot being a sufficiently snug fit for the volatile carrier that the parts of the volatile carrier projecting within the heating chamber are held in position by the snug contact between the slot and the volatile carrier. Also, a possible carrier holder can be a slot in the heating chamber ceiling, for use with a volatile carrier that is designed to be inserted downwardly through the slot and to hang from the edges of the slot from side tabs, a handle, or other parts of the volatile carrier that, because of their size or geometry, remain outside of the heating chamber, resting on outer surfaces of the ceiling.

Alternatively, the carrier holder may be an essentially open, either vertically disposed rack that leaves a mat or other volatile carrier held in the carrier holder directly exposed to hot gases rising in a convective flow from beneath.

Depending on the materials chosen and the volatile carrier temperatures desired, it is also possible to include a baffle spaced from and preferably located beneath the carrier holder and interposed between the fuel burner and a volatile carrier being held in the carrier holder. The baffle functions in part to mix hot gases from the fuel burner with air in the heating chamber prior to their reaching the volatile carrier. The result is believed to be a reduction of the tendency for a hot spot to form at a point on the volatile carrier directly above the fuel burner. Instead, the baffle causes a more even heating of the volatile carrier, whether the volatile carrier is heated solely by direct exposure to the hot gases or by a combination of direct exposure to hot gases and heat conducted through a sole plate.

The baffle can also function to more evenly distribute heat in another way. If the baffle is so located as to be heated by hot gases contacting the baffle from below, and if the carrier holder is spaced above the baffle, then the hot baffle serves as a radiant heater, supplementing heat delivered by a volatile carrier's direct contact with the hot gas flow by providing broadly distributed radiant heat to the volatile carrier.

Alternatively, the carrier holder can be in the form of an oven located within the heating chamber. "Oven" shall mean any substantially enclosed sub-chamber located within the heating chamber walls and made, preferably, of a heat-conductive material. The oven has oven walls and is positioned within the air-flow path. By this arrangement, the oven is heated by hot gases rising from the fuel burner. The oven holds a volatile carrier within the oven to receive heat radiating inwardly from the oven walls, an arrangement that provides for a more even heating of the volatile carrier. The oven preferably has openings sufficient to admit hot gases rising from the fuel burner so that they may directly contact the volatile carrier, and in any event has vents to allow volatile materials released from the volatile carrier to escape from the oven.

Although the fuel burner can be located beneath a heating chamber that has an open bottom, preferably the fuel burner is contained within the heating chamber itself. This arrangement contributes to the control and isolation of the convective flow of hot gases rising from the fuel burner and can also provide containment and protection for a burning flame. Thus, the walls of the heating chamber above the fuel burner can define the air flow path and limit the effects of breezes and other air movement external to the volatile dispenser.

It is sometimes desirable to reduce the temperature of the hot gases prior to their acting to heat the volatile carrier. To help achieve this, the heating chamber walls can be equipped with cooling vents communicating with the air outside of the heating chamber to cause unheated air to be drawn into the heating chamber by the passing flow of heated gases, to mix with and partially cool the hot gases from the fuel burner prior to their reaching the carrier holder. The cooling vents can be provided at any point in the air flow path, but preferably they are located at a point in the chamber walls at or above the level of the fuel burner but beneath the level at which a volatile carrier is held.

Although the fuel burner may burn fuel catalytically or otherwise without a flame and still fall within the breadth and scope of the invention, it is highly preferred that the fuel burner support a luminous flame positioned within the heating chamber and that the heating chamber walls include a light-transmitting portion, whether clear or translucent, that allows light from the flame to be visible to a user of the dispenser. This provides a ready means for a user to confirm that the fuel burner has been lit and continues to burn, and it also provides a use-up cue for the fuel. But the flame also provides light and aesthetic appeal, in much the same way that a citronella candle is valued in great part for its light. However, it is preferred that the carrier holder be positioned within a portion of the heating chamber whose walls are opaque or translucent so that the holder is at least not clearly visible through the chamber side walls. Walls will be deemed "visually obstructed" if they are opaque, translucent, or otherwise prevent the observation of distinct outlines of objects viewed therethrough.

The mats or other volatile carriers become exhausted and are designed to be replaced. To achieve this, preferably an insert slot communicates between the interior of the heating chamber and the exterior of the heated volatile dispenser, so that a fresh volatile carrier can be inserted through the insert slot to be held by the carrier holder. The insert slot can be in either the chamber walls or the ceiling of the heating chamber.

It can be important to prevent the use of a volatile carrier not intended for use with a particular volatile dispenser, to not mistakenly use, for example, a volatile carrier loaded with insecticide in a dispenser intended to supply perfume for indoor use. Therefore, it is preferred that the insert slot include keying structures that impart a cross-sectional profile to the insert slot that so restricts access thereto as to prevent the insertion of any volatile carrier not capable of presenting a non-interfering cross-sectional profile to the cross-sectional profile of the insert slot. This makes it easier to ensure that the only volatile carriers that will fit a given dispenser are those that are appropriate to a particular purpose or that are designed for use with the specific temperatures generated by the volatile dispenser. Also, the keying structures can be used to require that the volatile carrier be insertable only with a pre-determined side up or down. This can be important if the volatile carrier is, by way of example only, a gel cup that must be inserted so as to open upwardly. As examples, the keying structures can define a cross-sectional profile that includes either or both of angularly intersecting and curved sections.

Volatile carriers have a section treated or loaded (e.g. paper impregnated with insecticide) with the volatile material to be dispensed, and this section may itself be formed with a functionally required cross-sectional profile such as those just described.
Alternatively, the volatile carrier can include a handle in addition to a volatile-treated section, and the keying structures of the insert slot can be formed to present a non-interfering cross-sectional profile with respect to at least a portion of the volatile carrier and an interfering cross-sectional profile with respect to the handle for the volatile carrier.

In one embodiment, the heated volatile dispenser includes a fuel tank, containing fuel under pressure, and a fuel transfer route by which fuel can be transferred to the fuel burner in controlled amounts. Valves, constricted flow paths, wicks, pressure step-down controllers, or any other means may be used to control the delivery of pressurized fuel to the fuel burner in an amount sufficient to maintain combustion at a convenient level, and a variable valve may be used to allow a user to adjust the amount of fuel being burned. Preferably the fuel tank is replaceably removable. Ideally, the fuel tank contains fuel that burns as a pressurized gas, even though it may be a liquid at the tank pressures selected. Preferred gases include a gas selected from the group consisting of butane, isobutane, propane, compressed natural gas, and mixtures thereof.

An alternative and presently most preferred embodiment of the heated volatile dispenser of the invention is designed for use with a volatile carrier having a volatile-loaded section having a linearly extended volatile-releasing surface. The heated volatile dispenser includes a heat source that preferably is a fuel burner, the heat source generating a convective flow of hot gases. The heated volatile dispenser is used with a volatile carrier designed to hang or otherwise be positioned within the flow of hot gases in an orientation such that hot gas sweeps across the volatile-releasing surface, in a generally vertical direction but, in any event, in a direction generally parallel to the direction of linear extension of the volatile-releasing surface to release volatile therefrom. Preferably, the heated volatile dispenser includes a carrier holder that holds the volatile carrier within the flow of hot gases in that orientation. The heated volatile dispenser can also have any or all of the other features disclosed, above, with respect to the other embodiments, so long as those features are not inconsistent with the orientation of the volatile carrier just described.

A volatile carrier is used that has at least two volatile-releasing surfaces. The volatile carrier is then so designed, or the carrier holder, if present, then is designed, to hold the volatile carrier in an orientation such that hot gas sweeps across at least two of the volatile-releasing surfaces at the same time. The volatile carrier has front and back volatile-releasing surfaces. By way of example, only, a conventional mosquito mat has front and back surfaces, and the volatile carrier or the carrier holder can be designed such that the mat is held edge-on with respect to the flow of hot gases so that gas sweeps across both surfaces of the mat at the same time.

This arrangement has important advantages for the control of temperature across the volatile-releasing surfaces of the volatile carrier. The convective flow of hot gases above a sufficiently hot heater, and especially above a fuel burner that produces both heated air and gaseous combustion products, is fast compared to the conductive flow of heat through metal or other solid materials. Consequently, it is believed that the temperature of the hot gases does not drop much as the gases pass over the volatile-releasing surfaces. As a result, the volatile carrier is more evenly heated across its linear expanse so that volatiles are released more uniformly from the entire volatile-releasing surface. When the rate of volatile release from the volatile carrier drops sufficiently low that a fresh carrier is needed, the volatile from the exhausted carrier will have been more completely used than is the case when distinct, hotter and cooler regions are formed across the volatile-releasing surfaces.

When the heated volatile dispenser includes a carrier holder that is designed to be used with a volatile carrier having a linearly extended, volatile treated section having a leading edge to be presented toward the flow of hot gases, the carrier holder should preferably include a heat resistant edge guard that extends along the leading edge of a volatile carrier held in the carrier holder. The edge guard preferably extends the entire length of the leading edge. Alternatively, the edge guard can extend to protect only a portion of the leading edge that is exposed to the hottest area within the flow of hot gases, typically located at the center of the leading edge. An edge guard or a material will be understood to be "heat resistant" if it does not burn, char, or deform when subjected to the temperatures present at its location within a heated volatile dispenser when that dispenser is in use.

The edge guard protects the leading edge of the volatile carrier from heat directly radiating from a fuel burner and from the direct, edgeward impact of the flow of hot gases. Also, when the volatile carrier has at least two and preferably front and back volatile-releasing surfaces, the edge guard helps to split the flow of hot gases to direct the gases across the volatile-releasing surfaces. Either additionally or alternatively, a volatile carrier of the invention intended for such an edge-on orientation can be equipped with a carrier edge guard formed on or attached to the leading edge of the volatile carrier, itself. It is preferred that the edge guard, whether a part of the heated volatile dispenser or attached to the leading edge of the volatile carrier, include deflector vanes extending sidewardly with respect to the direction of linear extension of the volatile carrier's treated section to disrupt and mix the flow of hot gases before the gases contact the treated section.

In the presently most preferred embodiment of the invention, fuel burner is ventilated from beneath via a circumferentially extending open space surrounding the fuel burner, which space is vented to the ambient air. When a base is provided, located beneath the heating chamber, the base has a ventilation opening through which ambient air can pass to continue upwardly through the heating chamber. The fuel burner is so supported by the base in relation to the ventilation opening that the fuel burner is circumferentially ventilated from beneath. The preferred fuel burner in this arrangement employs a candle and preferably includes a candle cup having cup walls and a downwardly opening socket. The base then includes an attachment post to engage the socket and thus hold the candle cup. The heating chamber can include a light-transmitting chimney attached to the base.

Although the volatile dispenser of the invention (and preferably the embodiment just described) can be made with a base that can rest in a stable fashion on a flat supporting surface, it is also possible to provide for hanging the dispenser from a hook or other supporting structure. In that arrangement, the heated volatile dispenser includes a hanger by which the dispenser may be suspended from above. It is also then possible to so shape the underside of the base as to prevent the dispenser being supported in an upright orientation on a flat surface.

It is beneficial to provide for a candle that self-extinguishes, should the volatile dispenser tip over. To achieve this result, the fuel burner includes a candle contained within a candle cup, the candle cup having a floor and upright walls terminating in an open top and being made of a heat resistant material. The candle cup is fixedly positioned within the heating chamber, so that, should the volatile dispenser tip sidewardly while the candle is burning, the candle's heat contained within the candle cup will melt at least a portion of the candle's wax, allowing it to flow from the open top to starve the candle's wick of fuel, causing the candle to self-extinguish.

The invention also provides a method in accordance with claim 23.

A preferred method of the invention is disclosed for dispensing a volatile material from a volatile carrier having a volatile-loaded section having a linearly extended, volatile-releasing surface. The method includes the steps of providing a heat source, preferably a fuel burner, generating a flow of hot gases and holding the volatile carrier within the flow of hot gases in an orientation such that hot gas sweeps across the volatile-releasing surface, preferably in a direction generally parallel to the direction of linear extension of the volatile-releasing surface. The volatile carrier has both front and back volatile-releasing surfaces, and the step of holding the volatile carrier within the hot gases includes holding the volatile carrier in an orientation such that hot gas sweeps across both the front and back volatile releasing surfaces at the same time.

The invention also includes a volatile-dispensing volatile carrier suitable for use with a heated volatile dispenser having an insert slot through which the volatile carrier must be inserted for use, the insert slot having keying structures that impart a cross-sectional profile to the insert slot that departs from a straight cross-sectional profile and that so restricts access thereto as to prevent the insertion of any volatile carrier not capable of presenting a complementary cross-sectional profile. The volatile carrier of the invention includes a treated section having a cross-sectional profile complementary to that of the insert slot. The volatile. carrier also can have a handle having a cross-sectional profile that prevents the handle's entrance into the insert slot. Preferably the cross-sectional profile of the treated section includes curved or angularly intersecting sections, the latter including (without limitation) slots, prongs, ribs, and the like. Combinations of curved and angularly intersecting sections may also be used. Preferably the volatile carrier is a mat.

A kit is also disclosed for use with a heated volatile dispenser that employs a fuel burner to provide hot gases to heat and release a volatile material from a volatile carrier. The kit includes at least one volatile carrier, each volatile carrier bearing a selected amount of the volatile to be dispensed, and at least one fuel source for the fuel burner. The amount of fuel in a selected number of fuel sources is selected to be exhausted at substantially the same time that a selected amount of the volatile has been exhausted from at least one of the volatile carriers of the kit, whereby the exhausting of fuel provides a use up cue indicating that the selected amount of volatile has also been exhausted. Volatile is defined as being "exhausted" if the volatile has been released to the point that additional release of volatile is reduced to an amount or rate such that the desired effect to be accomplished by the volatile release is no longer achieved. A fuel source is defined as being exhausted at "substantially the same time" as the volatile is exhausted if the fuel burner extinguishes for lack of fuel when the volatile is either exhausted or when only that amount of volatile remains that a user is willing to discard.

Preferably, the kit includes only paired single fuel sources and single volatile carriers, with the volatile of a single volatile carrier being exhausted by the hot gases generated by the use of a single fuel source. However, it is also possible to achieve this benefit by requiring that two or more fuel sources be used before the volatile of a given carrier is exhausted. The goal is to have the easily perceived consumption of fuel serve as a use-up cue for the less easily detected consumption of the volatile of a volatile carrier. Preferably, the fuel source is a candle.

Preferably, the volatile carriers of the kit have a treated section that is linearly extended and holds volatile material to be dispensed, and the amount of volatile material held by a volatile carrier is selected to be exhausted by the hot gases generated by the use of a single fuel source when the volatile carrier is so positioned that the hot gases sweep over the linearly extended section. In an embodiment, the treated section has two sides and the amount of volatile material held by a volatile carrier is selected to be exhausted by the hot gases generated by the use of a single fuel source when the volatile carrier is so positioned that the hot gases sweep over the two sides of the linearly extended section. Ideally, the treated section has a front and a back and the volatile carrier has a leading edge. The amount of volatile material held by the treated section is selected to be exhausted by exposure to a flow of hot gases generated by the use of a single fuel source when the volatile carrier is so positioned that the flow of hot gases divides. with hot gases flowing to either side of the leading edge, to sweep over the sides of the treated section.

A preferred method of dispensing a volatile material from a volatile carrier includes a first step of providing a fuel source for the fuel burner having an amount of fuel selected to become exhausted (causing the fuel burner to extinguish) at the same time that the volatile of the volatile carrier is substantially exhausted. The effect of this step is to cause the extinguishing of the fuel burner to provide a use-up cue for the substantial exhaustion of volatile from the volatile carrier. The second step of the method is to light the fuel burner, with the volatile carrier in place. Preferably, the heated volatile dispenser used is of the sort in which fuel bums as a flame visible to a user of the dispenser. In that event, the steps of providing the fuel source and lighting the fuel burner include providing a visually observable use-up cue for the substantial exhaustion of volatile from the volatile carrier.

Also provided is a fuel burner useable with a heated volatile dispenser that has an attachment post for holding the fuel burner. The fuel burner includes a candle held within a open-topped candle cup. The candle cup has a cup floor, cup walls, and a downwardly opening socket extending downwardly beneath the cup floor and engageable on the attachment post. At least one cup support member extends downwardly beneath the cup floor at least as far as the socket extends. The at least one cup support member is at a location sufficiently remote from the socket and cumulatively extends circumferentially sufficiently to provide a stable support such that the candle cup can sit on a flat surface without tipping. Preferably, the cup support member extends around the entire margin of the cup floor. This arrangement is generally convenient for a user, but it is also important in the manufacture of the fuel burner in that it allows the candle cup to sit in a stable fashion on a flat conveyer belt or other materials handling surface. '

### BRIEF DESCRIPTION OF THE DRAWINGS

Fig. 1 is a perspective view of a heated volatile dispenser which uses a gas fuel source;
Fig. 2 is a top plan view thereof;
Fig. 3 is a vertical cross sectional view of the Fig. 1 arrangement;
Fig. 4 is an enlarged cross sectional view of the shut-off valve portion of Fig. 3;
Fig. 5 is a perspective view of another arrangement which uses a candle for fuel;
Fig. 6 is a cross sectional view of the arrangement of Fig. 5 taken along line 6-6 of Fig. 5;
Fig. 7 is a cross sectional view of the upper portion of a heated volatile dispenser like that of Fig. 1 or Fig. 5, albeit showing an alternative carrier holder having a conductive sole plate;
Fig. 8 is a cross sectional view of the upper portion of a heated volatile dispenser like that of Fig. 1 or Fig. 5, albeit showing another alternative volatile carrier holder in the form of an oven;
Fig. 9 is a cross sectional view of the upper portion of a heated volatile dispenser like that of Fig. 1 or Fig. 5, but in accordance with the present invention wherein a volatile carrier holder holds a volatile carrier in a vertical orientation;
Fig. 10 is a partial cross sectional view of the heated volatile dispenser of Fig. 9, albeit taken at an angle which is rotated 90 degrees relative to that of Fig. 9;
Fig. 11 is a perspective view of another volatile carrier of the invention having an edge guard;
Fig. 12 is a cross sectional view generally corresponding to Fig. 9, but showing how surfaces of the heating chamber ceiling can serve as the carrier holder;
Fig. 13 is a lower frontal perspective view of a form of volatile carrier that can be used when the top of the Fig. 9 embodiment is provided with the Fig. 15 inlet slot;
Fig. 14 is a lower frontal perspective view of another form of volatile carrier that can be used when the top of the Fig. 9 embodiment is provided with a wavy curve inlet slot; and
Fig. 15 is a top plan view of a dispenser having a ceiling with an insert slot suitable to receive the Fig. 13 volatile carrier.
Fig. 16 is a perspective view of an alternative embodiment of the heated volatile dispenser of the invention, including a candle and a volatile carrier, with portions of the chimney broken away.
Fig. 17 is a perspective view of the base of the heated volatile dispenser of Fig. 16, with the chimney removed and without a candle.
Fig. 18 is a cross sectional view of the heated volatile dispenser of Fig. 16, taken along sections lines 18--18 of Fig. 16.
Fig. 19 is a perspective view from beneath of a candle for use in the invention.
Fig. 20 is a cross sectional view of the candle of Fig., 19, taken along section lines 20--20 of Fig. 19.
Fig. 21 is a cross sectional view corresponding to that of Fig. 18 but with the heated volatile dispenser shown tipped on its side on a supporting surface.
Fig. 22 is a side elevation view of an alternative embodiment of the heated volatile dispenser of the invention.
Fig. 23 is a cross sectional view of the heated volatile dispenser of Fig. 22, the view otherwise generally corresponding to the view of Fig. 18.

### DETAILED DESCRIPTION OF THE INVENTION

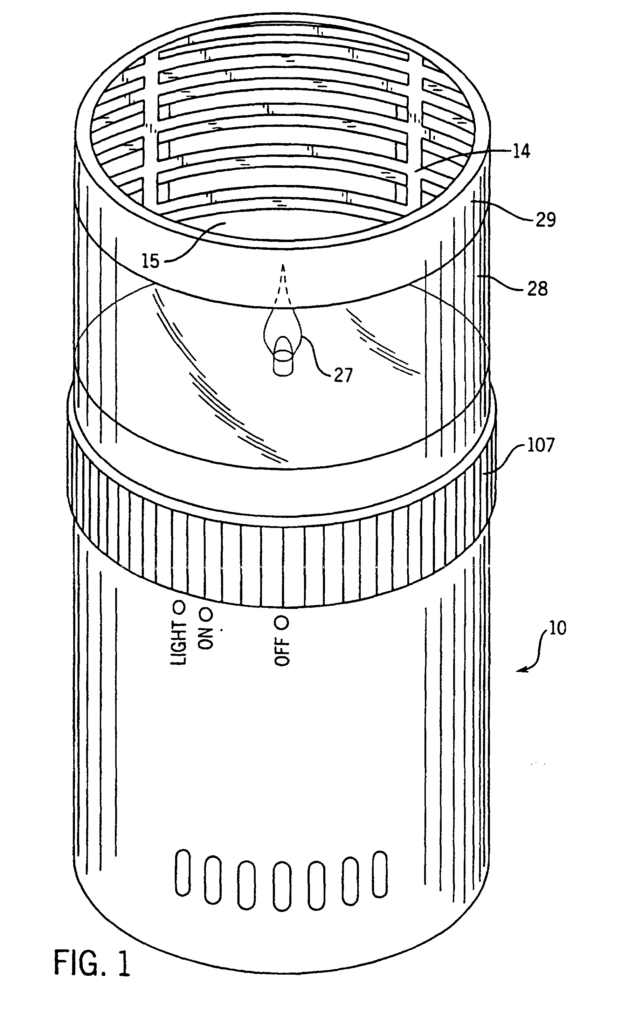

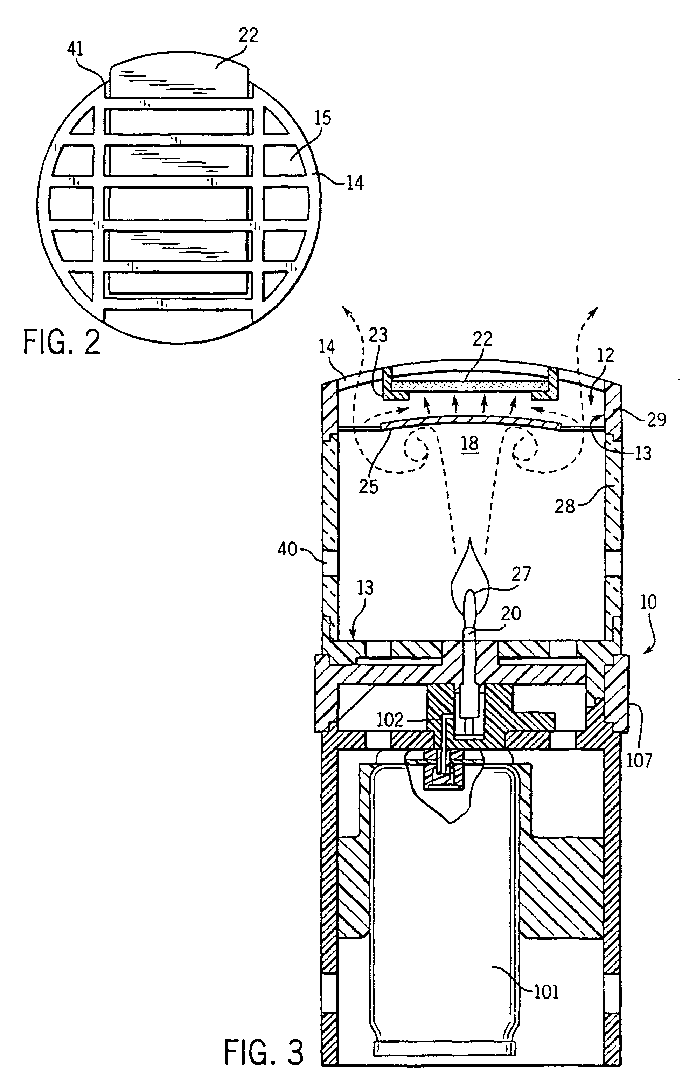

We turn first to the comparative example shown in Figs. 1-4. A dispenser, generally 10, encloses an internal heating chamber 12 having chamber side walls 13. There is also a chamber ceiling 14 that has exit vents 15.

The assembly includes a fuel burner 20. Fuel is supplied from a pressurized gas fuel source 101 through a fuel transfer route 102 by which fuel can be transferred to the fuel burner 20 in controlled amounts. Various types of valving and ignition systems can be used for this purpose (see e.g. U.S. patent 5,700,430).

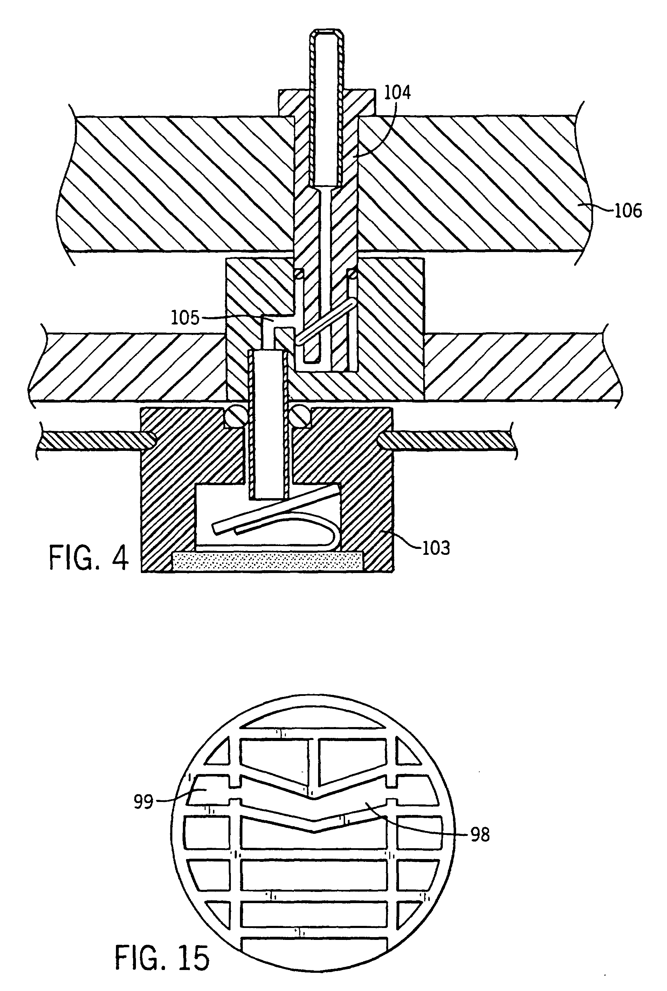

However, another option is depicted in Figs. 1 and 4. Rotation of outer ring 107 will cause rotation of inner ring 106, thereby rotating a lower extension therefrom, which acts as a valve to control the amount of fuel being provided. Various known ignition systems, not shown, can be incorporated as well.

The dispenser also includes a cellulosic mat-like carrier 22, preferably impregnated with an insect control ingredient, preferably an insecticide. The carrier is slid through insert slot 41 in the outer housing and rests on carrier holder 23. The carrier holder 23 is located above the fuel burner and within the heating chamber 12.

The walls of the chamber provide an air-flow path to guide hot gases from the fuel burner 20 past the carrier holder 23 to heat the carrier 22. This provides the direct exposure of the volatile carrier to the gases created by the flame 27.

Preferably there is also a baffle 25 interposed between the fuel burner 20 and the carrier 22. This creates turbulence in the region 18 so as to better mix gases prior to their reaching the carrier 22. The baffle 25 also acts as a radiant heater beneath the carrier holder 23.

There is a light transmitting transparent or translucent plastic portion 28 which allows light from the flame 27 to be visible to a user of the dispenser. Thus, the dispenser both dispenses the volatile and provides a light function. In this form, the fuel burner 20 is preferably also within the heating chamber 12.

There may also be a cooling vent 40 that permits air outside of the heating chamber 12 to enter the heating chamber and partially cool the hot gases prior to their reaching the carrier. Vent 40 is located above the level of the fuel burner.

The carrier holder 23 is positioned within a part 29 of the heating chamber 12 that is visually obstructed in that it is either opaque or translucent such that the carrier holder is not clearly visible through the chamber side walls. It is preferred that the wall portions 28 and 29 be permanently assembled together (e.g. sonic welded) so that the heating chamber remains continuously enclosed.

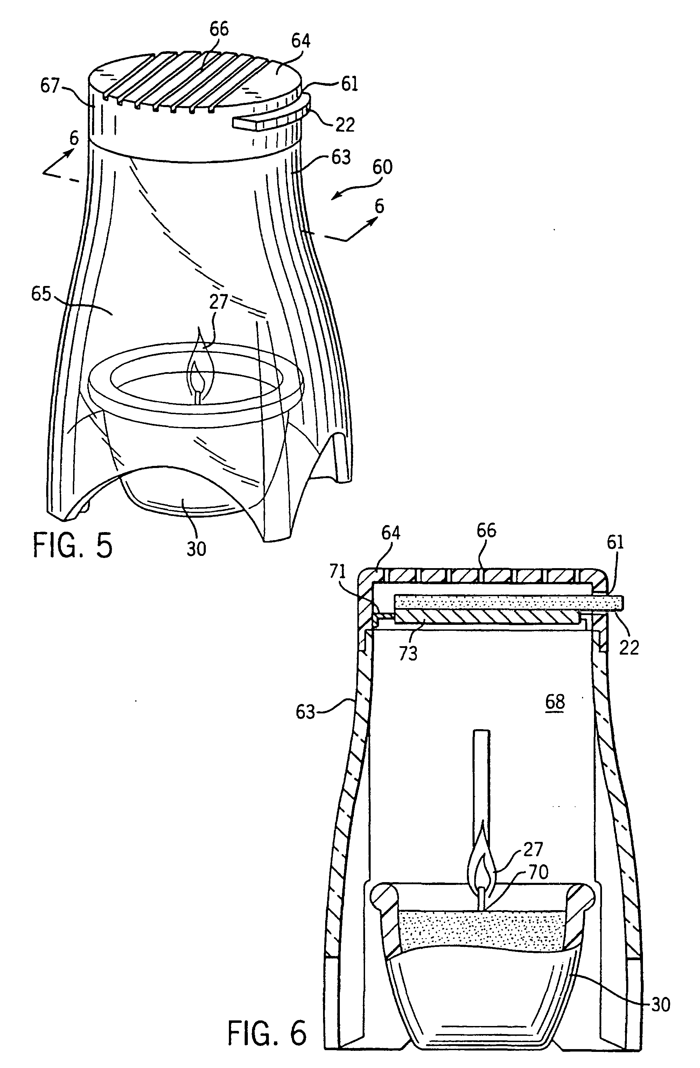

Turning next to the comparative example of Figs. 5-7 (generally 60), the fuel burner is now the wick of wax candle 30. There is a housing 65 with a cap 64 having vents 66. Side walls 63 help define the heating chamber. The volatile carrier 22 is inserted through slot 61 and in this case held on a sole plate 73 that is solid except for having spider leg radially peripheral attachments 71. The housing 65 can be lifted off the candle 30, the candle can be lit with a match, and the housing can be replaced to its Fig. 5 position.

In either case (the Fig. 1 or the Fig. 5 arrangement), the gases flow upward and ultimately around the volatile carrier before exiting. The gases will be sufficiently dispersed so as to provide desirable heating. The same flame which provides the heat source will also provide the light source.

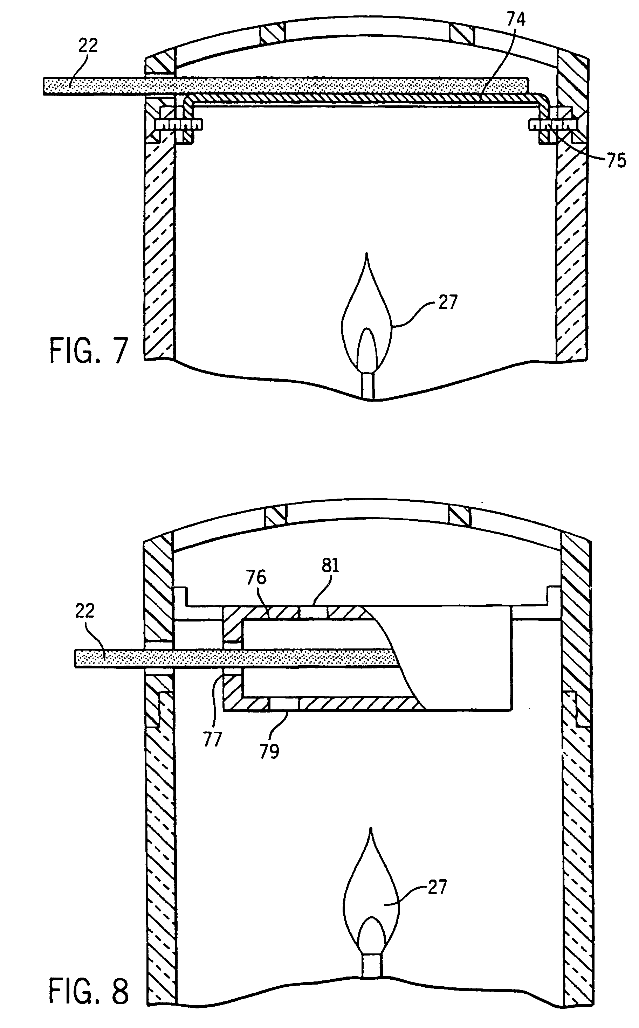

Turning now to Fig. 7, another version of the sole plate 74 has its ends alternatively supported in side brackets 75. The design is otherwise similar to the embodiment of Fig. 5.

As shown in Fig. 8, there is provided an oven (generally 76). It has a side slot 77 that is aligned with the outer insert slot so as to permit the carrier 22 to be inserted not only through the outer insert slot, but also in the oven. In use, the oven 76 has a sufficient heat capacity that it serves to maintain a more constant temperature within the oven than might otherwise be experienced at that location in the flow of hot, gaseous gases if, for example, the heat source were a flickering flame. Bottom hole 79 permits gases to readily enter the oven. Top hole 81 permits them to readily exit.

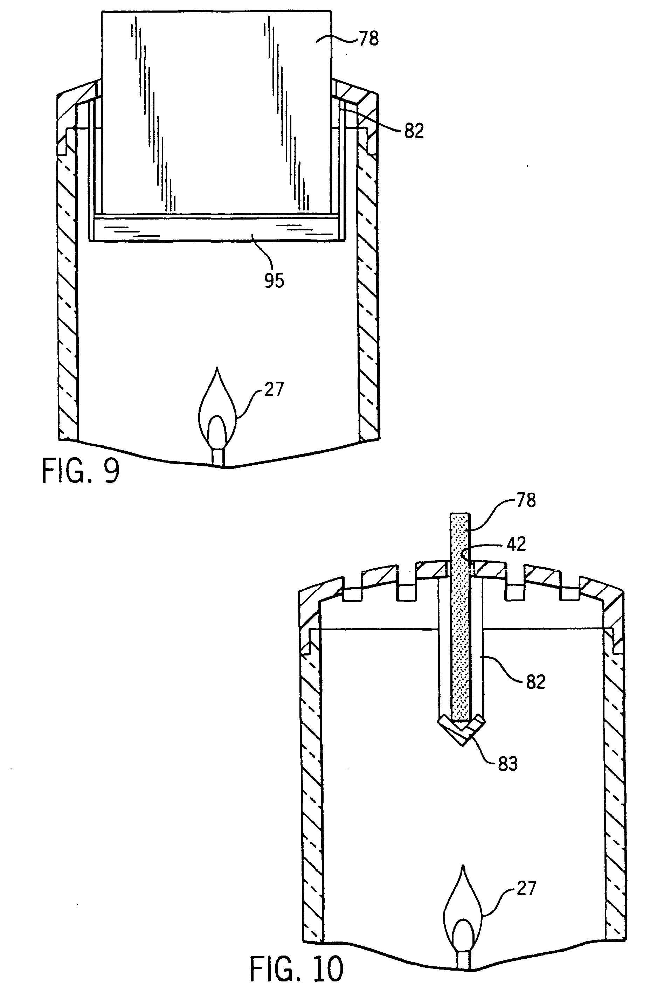

Figs. 9 and 10 depict embodiments of the invention. This arrangement has a generally vertically positioned carrier 78 inserted through an insert slot 42 and held by a carrier holder 82 having a protective guard 83 with side walls 95. This system has the advantage of exposing both sides of the carrier to roughly equivalent heat. The hot gas sweeps across the volatile-releasing surface in a direction generally parallel to the direction of linear extension of the volatile-releasing surfaces of the volatile carrier 22. Yet the downward edge of the volatile carrier is protected by protective guard 83 against undesirable overheating.

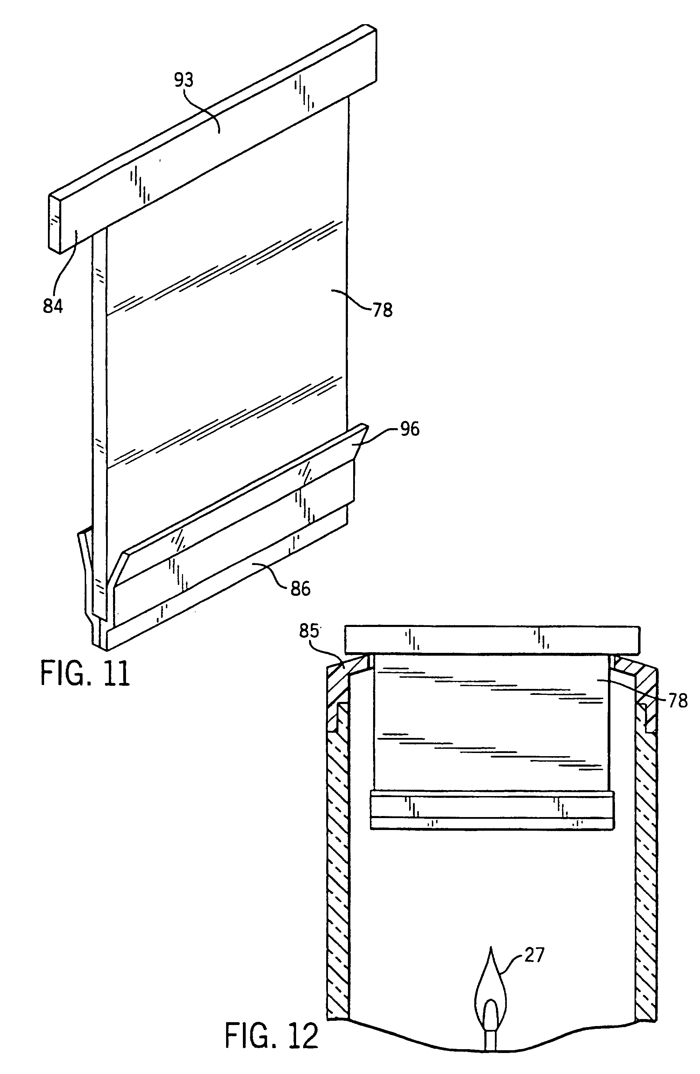

As shown in Fig. 11, the carrier 78 can be provided with a handle 93 and a heat resistant guard 86 positioned on a leading edge so as to be able to split the flow of hot gases when the carrier is held within the flow of hot gases. This again protects the treated section from edgeward impact of the hot gases. The guard preferably also has deflector vanes 96 extending sidewardly.

In these vertical forms, the carrier 78 is linearly extended and treated on both front and back sides.

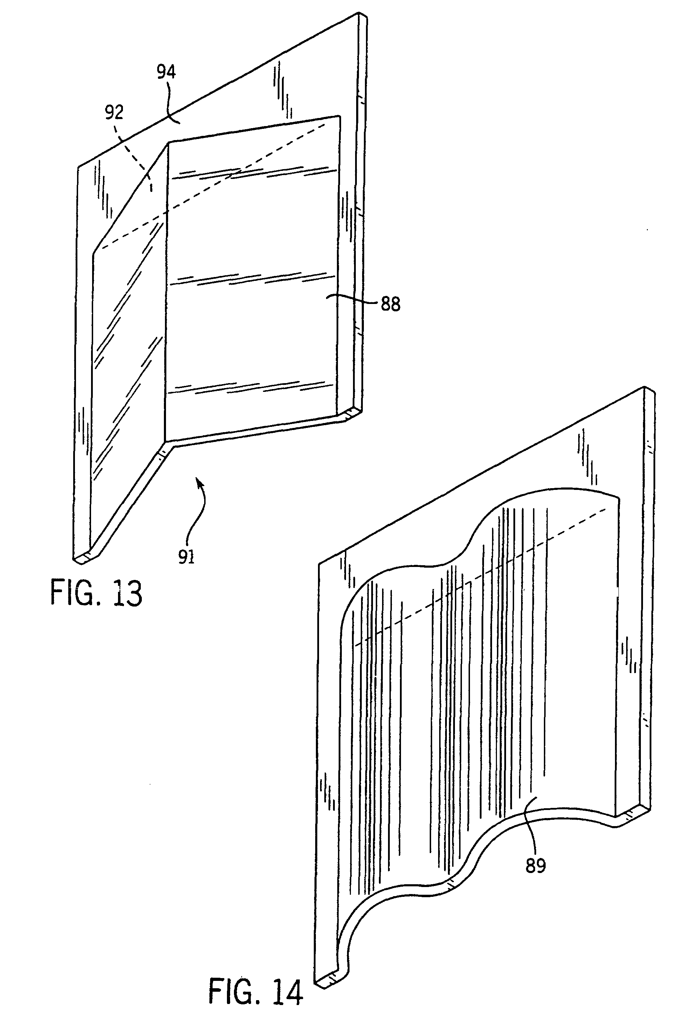

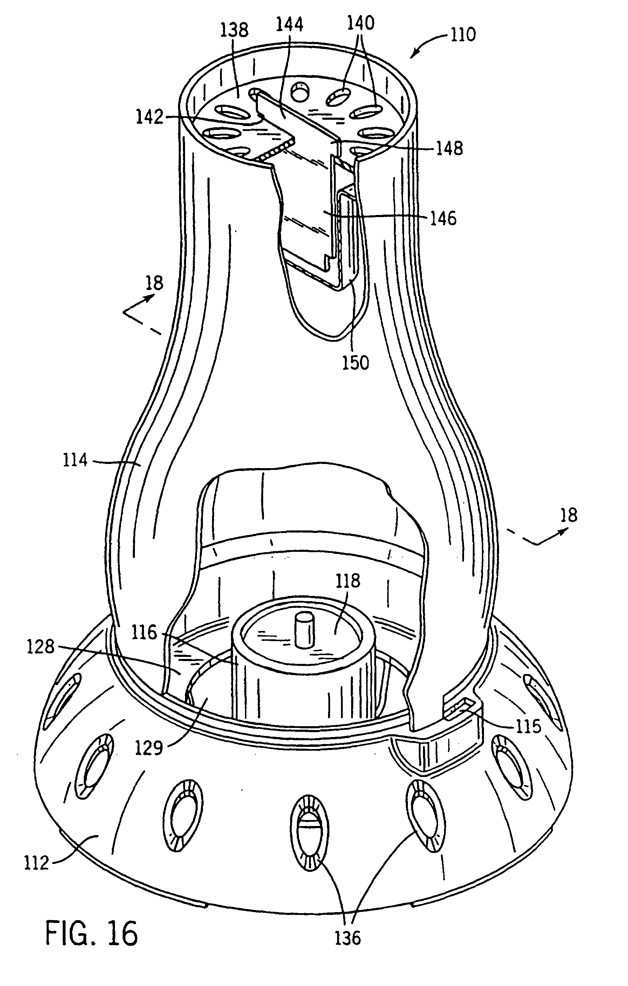

As best seen in Fig. 15, an insert slot 98 that is not simply rectangular can be formed in the ceiling of the dispenser. When used with a carrier such as carrier 88 of Fig. 13, the edge 91 presents a non-interfering cross-sectional profile with respect to the insert slot 98, while still allowing some venting via exits 99. The opposite surface from surface 92 shown presents an interfering cross-sectional profile preventing the handle 94 from falling through the insert slot.

If instead the carrier is carrier 89 as shown in Fig. 14, the Fig. 15 insert slot would then need to be a wavy line inlet. Thus, by using either form, the proper direction of the carrier can be controlled, and the public can be prevented from inserting mats into a given system that are not customized for use with that system.

In essence, this is a keying structure in which the cross-sectional profile of the insert slot must match with the cross-sectional profile of an inward end of the volatile carrier. The profile should depart from a rectangular slot, preferably using angularly intersecting and/or curved sections. Moreover, such a system is particularly useful in connection with horizontally extending carriers that have only one side treated with active.

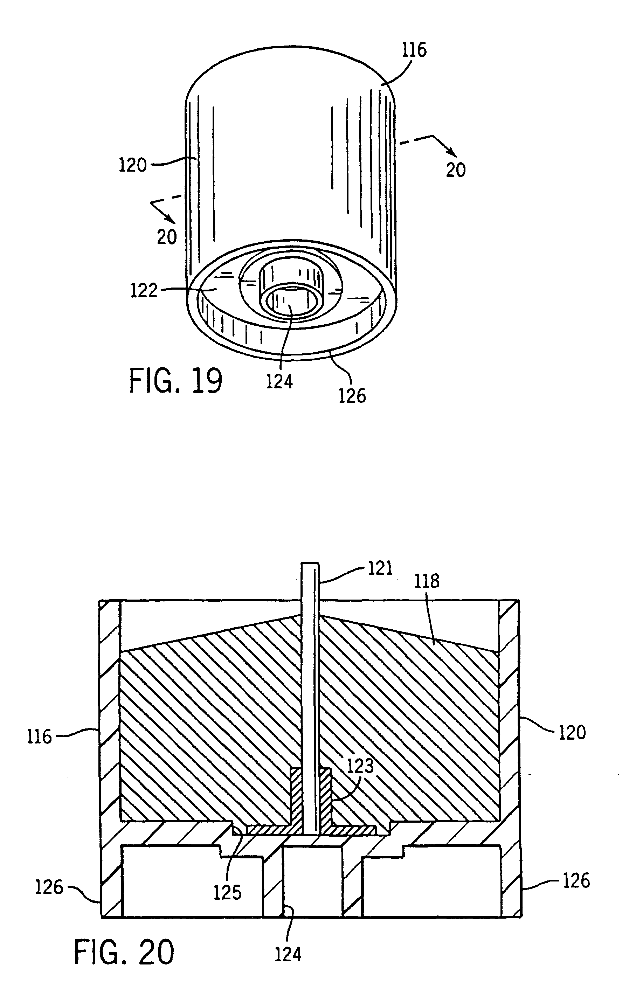

An alternative and presently most preferred embodiment of the heated volatile dispenser of the invention is shown generally at in Fig. 16. The dispenser 110 has a base 112 that supports a removable chimney 114, the chimney attaching to the base with locking tabs 113 formed on the lower edge of the chimney that mate with locking slots 115 formed in the base. The chimney can be made of glass or, preferably, a heat-resistive clear or (preferably) translucent plastic. A fire-resistant polycarbonate is the preferred chimney material, such as the material sold as V-O flame rated polycarbonate, available under the name "Makrolon® 6455" from Bayer Corporation. The base 112 supports a candle cup 116 positioned centrally within the chimney 114. The features of the candle cup 116 are best shown in Figs. 19 and 20.

The candle cup 116 is an open-topped, generally cylindrical cup that contains a wax candle 118. The candle cup 116 has cup walls 120 and a cup floor 122. Preferably the candle 118 has a wick 121 the bottom of which is held by a wick clip 123. Preferably the wick clip 123 is secured from slipping sidewardly on the cup floor 122. This can be accomplished in a variety of ways. For example, the wick clip 123 can simply be glued to the cup floor 122. Alternatively, a clip cup 125 can be formed as a central depression in the cup floor 122 having a diameter sized to receive the wick clip 123 but to restrain its sideways movement thereafter.

A centrally positioned, downwardly opening socket 124 extends downwardly from the underside of the cup floor 122. A cup support member 126 also extends downwardly from the cup floor 122 at least as far as the socket 124 extends and at locations remote from the socket. The cup support member 126 serves to facilitate manufacture and filling of the candle cup 116 by allowing the candle cup to sit upright on a conveyer belt or other surface without interference from the socket 124. The preferred cup support member 126 extends around the entire margin of the cup floor 122, as is best seen in Fig. 19. However, it will be appreciated by those skilled in the art that the cup support member need only be sufficiently remote from the socket and extend circumferentially sufficiently in one or more locations to provide a stable support such that the candle cup can sit on a flat surface without tipping. The candle cup 116 is made of a material sufficiently heat resistant as to be able to hold a burning candle therewithin without distorting or igniting. Once again, V-0 flame rated polycarbonate is a preferred material.

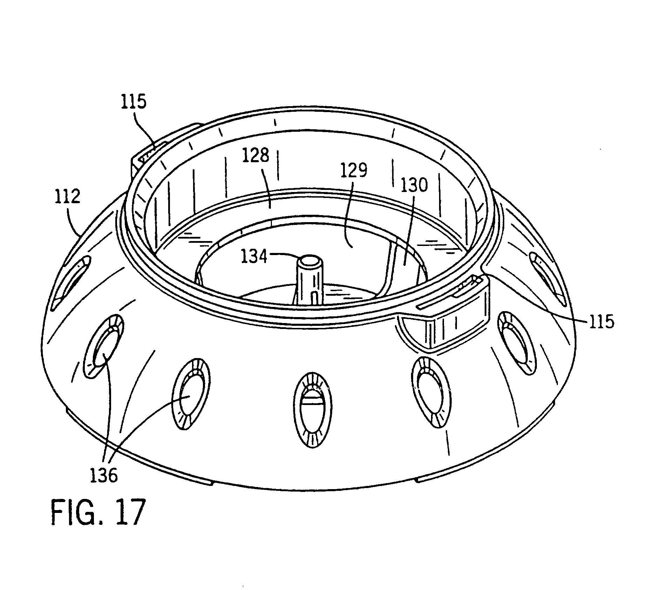

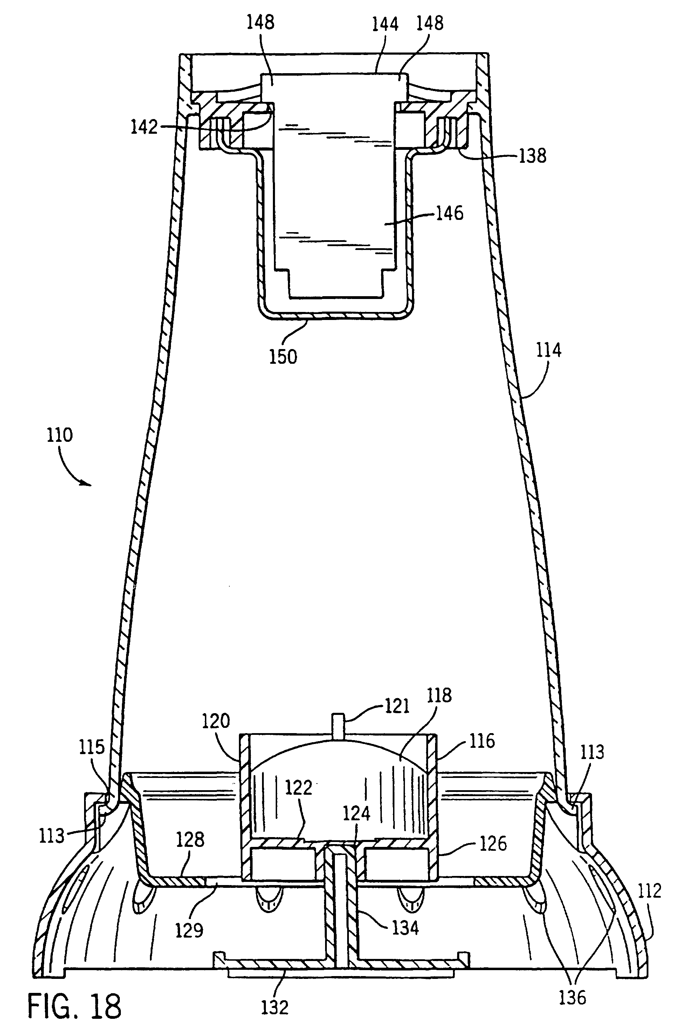

As can be best seen in Figs. 16-18, the base 112 has a base floor 128 that has a central ventilation opening 129 that is greater in diameter than the candle cup 116. Support elements 130 (seen in Fig. 16) extend downwardly from the underside of the base floor 128 and are attached to and support a wax catcher 132. The wax catcher 132 is a round, horizontally extending tray with low sides, the wax catcher having a diameter greater than that of the candle cup 116 so that any wax overflowing from the candle cup 116 lands on and is retained within the wax catcher. An attachment post 134 (seen in Figs. 17 and 18) extends upwardly from the wax catcher and is sized to be received within the socket 124 of the candle cup 116 in firmly gripping relation. By this arrangement, the candle cup 116 is held within the central ventilation opening 129 with a circumferentially extending open space surrounding the candle cup walls 120.

The base 112 has ventilation holes 136 that communicate between the ambient air and the space beneath the base floor 128. When the candle 118 is lit and the chimney 114 is in place on the base 112, a convective air flow is generated that pulls air in through the ventilation holes 136, upward under the candle cup 116 and through the open space of the ventilation opening 129 surrounding the candle cup walls 120, and on up the chimney. As a consequence, the candle 118 is ventilated from below the level of the candle cup, and consequently the candle cup floor 122 and walls 120 are cooled by the air flow. Furthermore, a sheath of cooler air appears to form, flowing upwardly within the chimney 114, surrounding the upward, centrally located flow of hot gases generated by the lit candle 118 and, in fact, tending to cause the hot gases to form a focused central area within the overall air flow that is hotter than the more stirred mix of gases and air experienced in a device otherwise similar but with air vents only at the periphery of the base floor 128. This pattern of air flow maintains both the candle cup 116 and the walls of the chimney 114 at a cooler temperature, while focusing a higher heat at the center of the area contained within the upper part of the chimney. This cooling effect helps to preserve the candle cup 116 and chimney 114 and make the chimney cooler to the touch while simultaneously establishing a hot area for driving off volatile material loaded on a substrate held in that area.

A ceiling 138 is positioned within the chimney 114 at its upper end. The ceiling 138 has ceiling vents 140 and an insert slot 142 that communicate between the interior of the chimney 114 and the outside air above the chimney. Hot gases flowing upwardly from the burning candle 118 can escape the chimney 114 through the ceiling vents 140. The insert slot 142 is sized to receive a volatile carrier, such as the mat 144 shown in Figure 16 and following. The preferred mat 144 is flat, having a linearly extended volatile bearing section 146 with sidewardly extending ears 148. The volatile bearing section 146 of the mat 144 is made small enough to be inserted from above into the insert slot 142, while the ears 148 are made too wide to slip through the insert slot. By this arrangement, the volatile bearing section 146 can be suspended within the chimney 114, with the mat 144 hanging by the ears 148, the insert slot 142 and upper surfaces of the ceiling 138 serving as a carrier holder, holding the volatile carrier--the mat 144--in a portion of the heating chamber that can be translucent, thus being a location that is visually obstructed.

A baffle strip 150, made of a heat-resistive material such as metal, is fastened to the under side of the ceiling 138, the baffle strip extending down one side of the volatile bearing section 146 of a mat 144 held beneath the ceiling, then sideways under the entire width of the volatile bearing section, and finally upwardly along the other side of the volatile bearing section. The baffle strip 150 serves to mix the flow of hot gases rising above the candle 118 and to protect the downwardly facing edge of the volatile bearing section 146 from the direct impact of the hottest gases rising from the candle.

The interior of the chimney 114 provides a heating chamber whose walls are defined by the sidewalls of the chimney. This heating chamber is vented to the outside air via the ceiling vents 140. The candle cup 116 provides a fuel burner with the candle 118 being its fuel source. The interior of the chimney 114 defines an air-flow path that guides the hot gases from the fuel burner past the mat 144, which is the volatile carrier of the device, to heat the mat by directly exposing it to the hot gases prior to their escape from the heating chamber into the surrounding air. The baffle strip 150 provides a baffle similar to the baffles described above in alternative embodiments of the heated volatile dispenser of the invention.

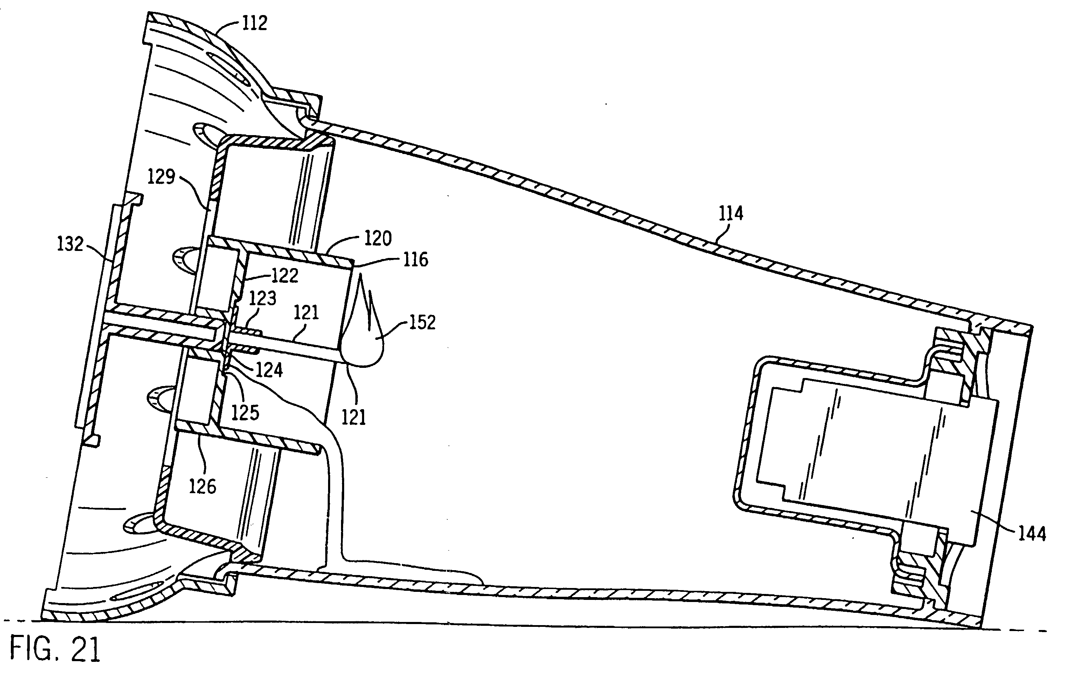

Preferably, the embodiment of the heated volatile dispenser shown generally at 110 is adapted to cause its candle 118 to self-extinguish if the dispenser tips over. The attachment post 134 is sized to be firmly gripped by the socket 124 when the candle cup 116 is installed in the base 112 to the extent necessary to retain the candle cup in place should the dispenser 110 tip over on its side, as is shown in Fig. 21. If the candle 118 is burning when the dispenser 110 tips over, any molten candle wax immediately drains from the now sidewardly opening candle cup 116. The flame 152 at the wick 121 continues to melt any remaining wax, which also drains from the candle cup 116, until the level of the wax has been so reduced as to no longer feed the wick. At that point, the flame 152 extinguishes. Although Fig. 20 illustrates a situation in which the candle cup 116 is sufficiently tipped as to be presented downwardly from the horizontal, the flame 152 will self-extinguish when tipped at any angle sufficient to allow molten wax to drain down to the level that the wick 121 becomes starved for fuel.

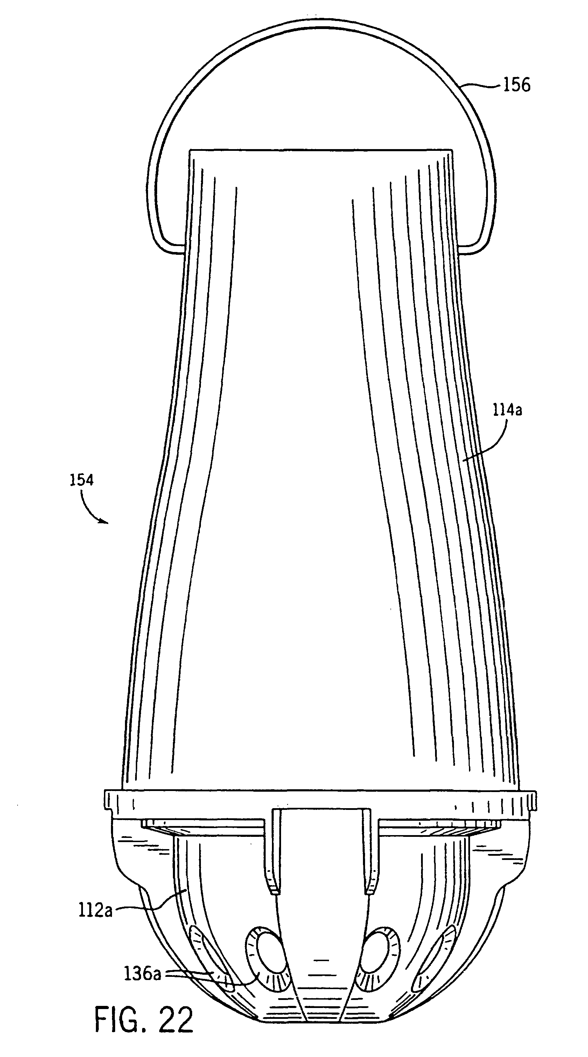

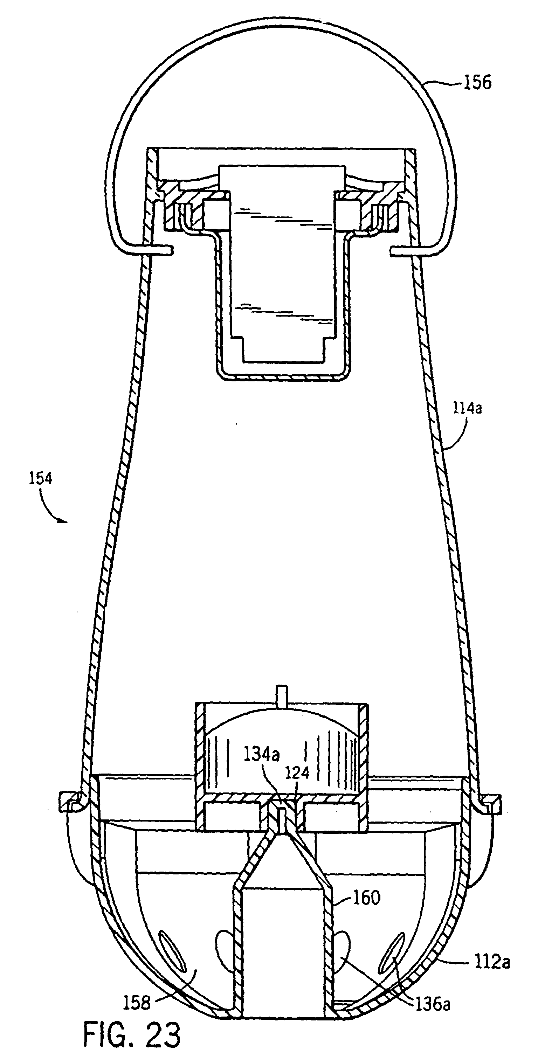

Fig. 22 is side elevational view (and Fig. 23 is a cross sectional view) of an alternative embodiment of the heated volatile dispenser of the invention, shown generally at 154. Volatile dispenser 154 is a modified form of the heated volatile dispenser 110 of Fig. 16. All parts of volatile dispenser 154 that directly correspond to parts of the volatile dispenser 110 are identified by the same reference numbers with the addition of the letter "a", without further discussion.

The volatile dispenser 154 differs from the volatile dispenser 110 in that dispenser 154 is designed to be hung from a hook or other overhead support (not shown). A hanger 156 capable of hanging from such a hook is attached to the upper part of the chimney 114a, preferably in freely turning relation to the chimney so that the weight of the dispenser 154 causes it to hang directly downwardly from the hook or other overhead support. This arrangement allows dispenser 154 to function generally in the same way as does dispenser 110 when dispenser 110 is resting on a horizontal surface.

The other differences between dispensers 154 and 110 all reside in the base 112a. The base 112a has a floor 158 that is downwardly curved at its center so as to discourage the use of dispenser 154 except by being hung.

The base 112a has an attachment post 134a that is sized to be received within the socket 124 of a candle cup 116. The attachment post 134a is located at the top of a central pedestal 160 that rises from the floor 158. When a candle cup 116 is mounted on the attachment post 134a, it is importantly advantageous for the cup walls 120 and cup floor 122 to be freely veritilated. Therefore, preferably the diameter of the central pedestal 160 is less than that of such a candle cup 116 for a distance beneath the bottom of the candle cup sufficient to allow for such ventilation. Preferably, the top of the pedestal 160 is an upwardly pointing cone or comparable, upwardly diminishing shape that terminates in the attachment post 134a, as is shown in Fig. 23. Also preferably, the central pedestal 160 is hollow and opens downwardly from the base 112a, allowing the dispenser 154 to be alternatively mounted on a stake or post (not shown) inserted into the central pedestal from beneath.

Base ventilation holes 136a are spaced around the base 112a at a level beneath that of the cup floor 122 of a candle cup 116 when it is mounted on the attachment post 134a. The base ventilation holes 126a communicate between the ambient air and the interior of the base. Thus, as in the dispenser 110, a candle cup 116 mounted on the attachment post 134a is held within a circumferentially extending open space. As in the dispenser 110, when a candle 118 is lit and the chimney 114a is in place on the base 112a, a convective air flow is generated that pulls air in through the ventilation holes 136a, upward under the candle cup 116 and through the open space surrounding the candle cup walls 120, and on up the chimney. As a consequence, the candle 118 is ventilated from below the level of the candle cup 116. Consequently the candle cup floor 122 and walls 120 are cooled, and the beneficial pattern of air flow discussed above with respect to the dispenser 110 is established.

A preferred embodiment of the kit of the invention, as disclosed above, includes at least one candle as a fuel source and at least one volatile carrier. The preferred candle is a candle 118, as already described, contained within a candle cup 116, and is made of paraffin wax with a preferred weight of from 15 to 20 grams and an especially preferred weight of from 16 to 17 grams. Ideally, the candle is made by the process of bonding small wax granules by simply forcefully pressing them in a compression mold. The technique is well known in the candle making art and produces candles of consistent dimensions and densities. The preferred candle, whether made by that or any other method, has a diameter of about 37 mm and an overall height at the candle's center of about 20 mm, the height tapering down to about 15 mm at the circumference of the candle. A candle of this size will burn for about 4 hours.

The preferred volatile carrier for the kit when used with the candle just described is made of conventional, fibrous mosquito mat material and preferably of a cellulosic, felted pulp mat material. The preferred mat weighs approximately 1 gram before being treated with ah insect control material, including the standard 5 to 7% moisture absorbed by such materials. Each such mat is treated with approximately 375 mg of d-cis/trans allethrin (or about 22% by weight of the mat) as a volatile insect control active ingredient. The heat from the preferred candle just described is sufficient to exhaust the d-cis/trans allethrin from the mat by the time the candle has been consumed, when the mat is positioned edge-on at approximately 9.5 cm above the candle in a location free of side drafts, such as is found within the heated volatile dispenser shown in Figs. 16 and following. The term "exhaust" has the meaning previously defined.

The various parts of the dispenser described above can be manufactured by conventional means from heat-resistant plastics, metal, glass, and the like. The volatile carriers disclosed can be made using conventional methods and materials well known in the art, such as those used for making conventional mosquito mats, volatile gel carriers, volatile-containing polymers, and the like.

The preceding description is merely of preferred embodiments of the invention. One skilled in the art will readily apprehend alternative embodiments that nevertheless fall within the scope and breadth of the invention. Thus, the claims should be looked to in order to understand the full scope of the invention.

### INDUSTRIAL APPLICABILITY

Heated volatile dispensers and volatile carriers, and methods of using them, are described. They are useful in the practical control of insects and other pests and in air scenting.

## Claims

1. A heated volatile dispenser (110) for dispensing volatile materials from a volatile carrier, comprising:
a. a volatile carrier (144) having a front and a back exposed major surfaces to release volatile material;
b. an enclosed heating chamber (114) having chamber walls and being vented to the outside air;
c. a fuel burner (118); and
d. an air flow path (114,140) to guide hot gasses generated by the fuel burner (118) past the volatile carrier (144) held within the heating chamber (114,140) to heat the volatile carrier (144) by the direct exposure of the volatile carrier (144) to the hot gasses, the airflow path then directing the hot gasses to escape from the dispenser to the outside air;
**characterized in that**
the volatile carrier (144) is held within the heating chamber (114) in an orientation such that the hot gasses sweep across the exposed major surfaces in a generally vertical direction generally parallel to the direction of linear extension of the exposed surfaces of the volatile carrier (144) the orientation exposing both front and back sides of the volatile carrier (144) to the hot gasses in the airflow path (114, 140).

2. A dispenser according to claim 1, wherein the heating chamber has a ceiling (138) with an insertion slot for the volatile carrier (144).

3. A dispenser according to claim 2, wherein the volatile carrier (78) has a heat resistant edge guard (86) protecting its lower edge against undesired overheating.

4. A dispenser according to claim 1, wherein the volatile carrier (78) is suspended in a carrier holder (82).

5. A dispenser according to claim 4, wherein the carrier holder (82) includes a heat resistant edge guard (83) protecting its lower edge against undesired overheating.

6. A dispenser according to claim 2, wherein said volatile carrier (78,144) has at its upper edge a portion (93) of interfering cross section with respect to said slot, whereby the carrier hangs in the heating chamber by the said portion of interfering cross section (93).

7. A heated volatile dispenser according to claim 6, wherein the insert slot includes keying structures that impart a cross-sectional profile to the insert slot that so restricts access thereto as to prevent the insertion through the slot of any volatile carrier (88, 89) not capable of presenting a non-interfering cross-sectional profile to the cross-sectional profile of the insert slot.

8. A heated volatile dispenser according to claim 6, wherein the keying structures define a cross-sectional profile selected from the group consisting of angularly intersecting and curved sections.

9. A heated volatile dispenser according to any preceding claim, wherein the fuel burner (118) supports a flame positioned within the heating chamber and the heating chamber walls include a light-transmitting portion (114) that allows light from the flame to be visible to a user of the dispenser through the light-transmitting portion (114).

10. A heated volatile dispenser according to claim 9, wherein
a. the walls of a part of the heating chamber are selected from the group consisting of opaque and translucent so as to be visually obstructed, and
b. the location at which a volatile carrier is held within the heating chamber is within the visually obstructed part of the heating chamber.

11. A heated volatile dispenser according to any preceding claim, wherein the fuel burner (118) bums fuel selected from the group consisting of a candle, a burnable solid, a pressurized gas, a combustible liquid, and a gelled combustible liquid.

12. A heated volatile dispenser according to claim 1, wherein the fuel burner (118) is ventilated from beneath via a circumferentially extending open space (129) surrounding the fuel burner (118), which space is vented to the ambient air.

13. A heated volatile dispenser according to any preceding claim, including a base (112) located beneath the heating chamber (114), the base (112) having a ventilation opening (136,129) through which ambient air can pass to continue upwardly through the heating chamber (114) and wherein the fuel burner (118) is so supported by the base in relation to the ventilation opening that the fuel burner is circumferentially ventilated from beneath.

14. A heated volatile dispenser according to any preceding claim, wherein the fuel burner employs a candle.

15. A heated volatile dispenser according to claim 13, wherein the fuel burner (118) includes a candle cup (116) having cup walls and a downwardly opening socket, and the base includes an attachment post to engage the socket and thus hold the candle cup (116).

16. A heated volatile dispenser according to claim 13, wherein the heating chamber includes a light-transmitting chimney attached to the base.

17. A heated volatile dispenser according to claim 13 including a hanger (156) by which the dispenser may be suspended from above.

18. A heated volatile dispenser according to claim 17, wherein the underside of the base (112a) is so shaped as to prevent the dispenser being supported in an upright orientation on a flat surface.

19. A heated volatile dispenser according to claim 1, wherein
a. the fuel burner (118) includes a candle contained within a candle cup (116), the candle cup (116) having a floor (122) and upright walls (120) terminating in an open top and being made of a heat resistant material, and
b. the candle cup (116) is fixedly positioned within the heating chamber (114), so that, should the volatile dispenser tip sidewardly while the candle is burning, the candle's heat contained within the candle cup will melt at least a portion of the candle's wax, allowing it to flow from the open top to starve the candle's wick of fuel, causing the candle to self-extinguish.

20. A volatile dispenser according to claim 3, wherein the edge guard has deflector vanes extending sidewardly with respect to the direction of surface of the volatile carrier (78).

21. A heated volatile dispenser according to claim 1, wherein the fuel burner is selected from the group consisting of a candle, a solidified combustible liquid, a burnable solid, a catalytic heater, a pressurized gas burner, and a wick that is fuelled with a combustible liquid.

22. A dispenser according to any preceding claim, wherein said volatile carrier (144) bears a selected amount of the volatile to be dispensed and there is provided a fuel source for the fuel burner (118), the amount of fuel in the fuel source being selected to be exhausted at substantially the same time that a selected amount of the volatile has been exhausted from the volatile carrier (144), whereby the exhausting of fuel provides a use up cue indicating that the selected amount of volatile has also been exhausted.

23. A method for dispensing ingredients volatilizable by application of heat, the method comprising the steps of:
a. providing a heated volatile dispenser (110) having:
i. a volatile carrier (144) with a front and a back exposed major surfaces to release volatile material;
ii. an enclosed heating chamber (114) having chamber walls and being vented to the outside air;
iii. a fuel burner (118); and
iv. an air-flow path (114,140) to guide hot gasses from the fuel burner (118) past a volatile carrier held within the heating chamber to heat the volatile carrier by the direct exposure of the volatile carrier (144) to the hot gasses, the air flow path then directing the hot gasses to escape from the dispenser to the outside air;
b. positioning the volatile carrier (144) loaded with volatile ingredients in the heating chamber (114) in an orientation such that the hot gasses sweep across the exposed major surface in a generally vertical direction generally parallel to the direction of linear extension of the exposed surfaces of the volatile carrier (144) the orientation exposing both front and back sides of the volatile carrier (144) to the flow of hot gasses;
c. igniting the fuel at the burner (118); and
d. allowing the ingredients thus volatilized from the volatile carrier (144) to be vented from the dispenser.

24. A method according to claim 23 further including the step of providing a fuel source for the fuel burner (118) having an amount of fuel selected to become exhausted, causing the fuel burner to extinguish, at the same time that the volatile ingredients on the volatile carrier are substantially exhausted so that the extinguishing of the fuel burner is a use-up cue for the substantial exhaustion of volatile ingredients from the volatile carrier.

## Patentansprüche

1. Beheizte Vorrichtung (110) zur Abgabe flüchtiger Stoffe von einem Träger für flüchtige Stoffe mit:
a. einem Träger (144) für flüchtigen Stoff mit einer vorderen und einer hinteren, frei liegenden Hauptfläche zur Abgabe von flüchtigem Stoff;
b. einer umschlossenen Heizkammer (114) mit Kammerwänden, die zur Umluft gelüftet ist;
c. einem Brennstoffbrenner (118); und
d. einem Luftströmungsweg (114, 140), mit dem vom Brennstoffbrenner (118) erzeugte heiße Gase am in der Heizkammer (114, 140) befindlichen Träger (144) vorbei leitbar sind, um den Träger (144) den heißen Gasen direkt auszusetzen und so zu erwärmen, wobei der Luftströmungsweg dann die heißen Gase aus der Abgabevorrichtung hinaus in die Umluft leitet;
**dadurch gekennzeichnet, dass**
der Träger (144) für flüchtige Stoffe in der Heizkammer (114) in einer solchen Orientierung gehaltert ist, dass die heißen Gase in einer allgemein vertikalen Richtung, die allgemein parallel zur Richtung der gradlinigen Ausdehnung der frei liegenden Flächen des Trägers verläuft, über dessen frei liegende Hauptflächen streichen, wobei diese Orientierung sowohl die Vorder- als auch die Rückseite des Trägers (144) den heißen Gasen auf dem Luftströmungsweg (114, 140) aussetzt.

2. Abgabevorrichtung nach Anspruch 1, deren Heizkammer eine Decke (138) mit einem Einführschlitz für den Träger (144) aufweist.

3. Abgabevorrichtung nach Anspruch 2, deren Träger (78) einen wärmefesten Kantenschutz (86) aufweist, der dessen untere Kante vor unerwünschter Überhitzung schützt.

4. Abgabevorrichtung nach Anspruch 1, deren Träger (78) in einem Trägerhalter (82) aufgehängt ist.

5. Abgabevorrichtung nach Anspruch 4, deren Trägerhalter (82) einen wärmefesten Kantenschutz (83) aufweist, der dessen untere Kante vor unerwünschter Überhitzung schützt.

6. Abgabevorrichtung nach Anspruch 2, deren Träger (78) an seiner oberen Kante einen Teil (93) mit einem Querschnitt hat, der nicht kompatibel ist mit dem des Schlitzes, wobei der Träger an diesem Teil (93) nicht kompatiblen Querschnitts in die Heizkammer hinabhängt.

7. Beheizte Abgabevorrichtung für flüchtige Stoffe nach Anspruch 6, bei der der Einführschlitz Schlüsselelemente aufweist, die ihm ein Querschnittsprofil erteilen, das den Zugang zum Schlitz einschränkt, um das Einführen eines Trägers (88, 89) für flüchtige Stoffe in den Einführschlitz zu verhindern, der ein mit dem des Einführschlitzes nicht kompatibles Querschnittsprofil hat.

8. Beheizte Abgabevorrichtung für flüchtige Stoffe nach Anspruch 6, bei der die Schlüsselelemente ein Querschnittsprofil bilden, das ausgewählt ist aus der Gruppe aus winklig sich schneidenden und gekrümmten Abschnitten.

9. Beheizte Abgabevorrichtung für flüchtige Stoffe nach einem der vorgehenden Ansprüche, deren Brennstoffbrenner (118) eine Flamme in der Heizkammer stützt und die Heizkammerwand einen lichtdurchlässigen Teil (114) aufweist, durch den hindurch ein Benutzer der Abgabevorrichtung das Licht der Flamme sehen kann.

10. Beheizte Abgabevorrichtung für flüchtige Stoffe nach Anspruch 9, bei der
a. die Wände eines Teils der Heizkammer aus der Gruppe opak und durchscheinend gewählt sind, um nicht einsehbar zu sein, und
b. der Ort, an dem der Träger in de Heizkammer gehaltert ist, sich im nicht einsehbaren Teil der Heizkammer befindet.

11. Beheizte Abgabevorrichtung für flüchtige Stoffe nach einem der vorgehenden Ansprüche, deren Brennstoffbrenner einen Brennstoff aus der Gruppe verbrennt, die aus einer Kerze, einem brennbaren Feststoff, einem Druckgas, einer brennbaren Flüssigkeit und einer gelierten brennbaren Flüssigkeit besteht.

12. Beheizte Abgabevorrichtung für flüchtige Stoffe nach Anspruch 1, deren Brennstoffbrenner (118) durch einen in Umfangsrichtung sich erstreckenden offenen Raum (129) von unten gelüftet wird, der den Brennstoffbrenner (118) umgibt und zur Umluft gelüftet ist.

13. Beheizte Abgabevorrichtung für flüchtige Stoffe nach einem der vorgehenden Ansprüche mit einem Fuß (112) unter der Heizkammer (114), der eine Lüftungsöffnung (136,129) enthält, durch die Umluft eintreten und weiter aufwärts durch die Heizkammer (114) strömen kann, wobei der Brennstoffbrenner (118) vom Fuß relativ zur Lüftungsöffnung so gehaltert wird, dass er von unten entlang seines Umfangs gelüftet wird.

14. Beheizte Abgabevorrichtung für flüchtige Stoffe nach einem der vorgehenden Ansprüche, deren Brennstoffbrenner eine Kerze verwendet.

15. Beheizte Abgabevorrichtung für flüchtige Stoffe nach Anspruch 13, deren Brennstoffbrenner (118) einen Kerzenbecher (116) mit Becherwänden und einer nach unten offenen Fassung aufweist, wobei der Fuß einen Aufsetzpfosten enthält, der in die Fassung eingreift und so den Kerzenbecher (116) haltert.

16. Beheizte Abgabevorrichtung für flüchtige Stoffe nach Anspruch 13, deren Heizkammer einen lichtdurchlässigen Kamin aufweist, der an den Fuß angesetzt ist.

17. Beheizte Abgabevorrichtung für flüchtige Stoffe nach Anspruch 13 mit einer Aufhängeeinrichtung (156), mit der die Abgabevorrichtung von oben aufhängbar ist.

18. Beheizte Abgabevorrichtung für flüchtige Stoffe nach Anspruch 17, deren Fuß (112a) auf der Unterseite so geformt ist, dass sich die Abgabevorrichtung nicht auf eine ebene Fläche aufstellen lässt.

19. Beheizte Abgabevorrichtung für flüchtige Stoffe nach Anspruch 1, bei der
a. der Brennstoffbrenner (118) eine Kerze in einem Kerzenbecher (116) aufweist, der einen Boden (122) und aufrechte Wände (120) hat, die nach oben offen auslaufen und aus einem wärmefesten Werkstoff gefertigt sind; und
b. der Kerzenbecher (116) in fester Lage in der Heizkammer (114) so angeordnet ist, dass, wenn die Abgabevorrichtung bei brennender Kerze seitlich kippt, die Wärme der Kerze im Kerzenbecher mindestens einen Teil des Kerzenwachses schmilzt und dieses ausfließen lässt, so dass der Kerzendocht keinen Brennstoff mehr erhält und von selbst erlischt.

20. Beheizte Abgabevorrichtung für flüchtige Stoffe nach Anspruch 3, bei der der Kantenschutz Ablenkflügel aufweist, die sich bezüglich der Richtung der Oberfläche des Trägers (78) seitlich erstrecken.

21. Beheizte Abgabevorrichtung für flüchtige Stoffe nach Anspruch 1, bei der der Brennstoffbrenner gewählt ist aus der Gruppe, die besteht aus einer Kerze, einer verfestigten brennbaren Flüssikeit, einem brennbaren Feststoff, einer katalytischen Heizeinrichtung, einem Druckgasbrenner und einem Docht, der mit einer brennabren Flüssigkeit gefüllt ist.

22. Abgabevorrichtung nach einem vorgehenden Anspruch, bei der der Träger (144) für den abzugebenden flüchtigen Stoff eine gewählte Menge desselben enthält und für den Brennstoffbrenner (118) eine Brennstoffquelle bereit gestellt ist, wobei die Brennstoffmenge in der Brennstoffquelle so gewählt ist, dass sie im wesentlichen gleichzeitig mit einer gewählten Menge des flüchtigen Stoffs im Träger (144) aufgebraucht ist, so dass das Erschöpfen des Brennstoffs einen Aufbrauchshinweis darstellt dahingehend, dass auch die gewählte Menge des flüchtigen Stoffs erschöpft ist.

23. Verfahren zur Abgabe von Wirkstoffen, die durch Aufbringen von Wärme verflüchtigbar sind, mit folgenden Schritten:
a. Bereitstellen einer beheizten Abgabevorrichtung (110) für flüchtige Stoffe mit:
i. einem Träger (144) für flüchtigen Stoff mit einer vorderen und einer hinteren Hauptseite, an denen flüchtiger Stoff freisetzbar ist;
ii. einer umschlossenen Heizkammer (114) mit Kammerwänden, die zur Umluft gelüftet ist;
iii. einem Brennstoffbrenner (118); und
iv. einem Luftströmungsweg (114,140), mit dem heiße Gease vom Brennstoffbrenner (118) kommend an einem Träger für flüchtigen Stoff vorbei leitbar sind, der in der Heizkammer gehaltert ist, um durch direkte Exposition von den heißen Gasen erwärmt zu werden, wobei der Luftsrömungsweg dann die heißen Gase aus der Abgabevorrichtung hinaus an die Umluft leitet;
b. Anordnen des mit flüchtigen Wirkstoffen beschickten Trägers (144) in der Heizkammer (114) in einer solchen Orientierung, dass die heißen Gase über die frei liegende Hauptfläche in einer allgemein vertikalen Richtung streichen, die parallel zur Richtung der gradlinigen Ausdehnung der frei liegenden Flächen des Trägers (144) verläuft, wobei diese Orientierung sowohl die Vorder- als auch die Rückseite des Trägers (144) der Strömung heißer Gase aussetzt;
c. Entzünden des Brennstoffs am Brenner (118); und
d. Entweichenlassen der so flüchtig gemachten und vom Träger (144) abgegebenen Wirkstoffe aus der Abgabevorrichtung hinaus an die Umluft.

24. Verfahren nach Anspruch 23, weiterhin mit dem Schritt des Bereitstellens einer Brennstoffquelle für den Brennstoffbrenner (118), die eine solche Brennstoffmenge aufweist, dass sie aufgebraucht ist, so dass der Brennstoffbrenner erlischt, wenn auch die flüchtigen Wirkstoffe auf dem Träger derselben im wesentlichen aufgebraucht sind, so dass das Erlöschen des Brennstoffbrenners ein Aufbrauchshinweis ist, der anzeigt, dass die flüchtigen Wirkstoffe vom Träger im wesentlichen erschöpft sind.

## Revendications

1. Distributeur de produits volatils chauffés (110) pour distribuer des matériaux volatils à partir d'une réserve de produit volatil, comprenant :
a. une réserve de produit volatil (144) ayant des surfaces majeures exposées, une avant et une arrière, pour libérer le matériau volatil ;
b. une chambre de chauffage fermée (114) ayant des parois de chambre et étant ventilée vers l'air extérieur ;
c. un brûleur de combustible (118) ; et
d. un passage d'écoulement d'air (114, 140) pour guider des gaz chauds générés par le brûleur de combustible (118) au-delà de la réserve de produit volatil (144) maintenue à l'intérieur de la chambre de chauffage (114, 140) pour chauffer la réserve de produit volatil (144) par l'intermédiaire de l'exposition directe de la réserve de produit volatil (144) aux gaz chauds, le passage d'écoulement d'air dirigeant alors les gaz chauds pour être évacués à partir du distributeur vers l'air extérieur ;
**caractérisé en ce que**
la réserve de produit volatil (144) est maintenue à l'intérieur de la chambre de chauffage (114) dans une orientation telle que les gaz chauds passent le long des surfaces majeures exposées dans une direction généralement verticale généralement parallèle à la direction de prolongement linéaire des surfaces exposées de la réserve de produit volatil (144), l'orientation exposant à la fois les côtés avant et arrière de la réserve de produit volatil (144) aux gaz chauds dans le passage d'écoulement d'air (114, 140).

2. Distributeur selon la revendication 1, dans lequel la chambre de chauffage a un plafond (138) avec une fente d'insertion pour la réserve de produit volatil (144).

3. Distributeur selon la revendication 2, dans lequel la réserve de produit volatil (78) a un dispositif de protection de bord résistant à la chaleur (86) protégeant son bord inférieur contre une surchauffe indésirable.

4. Distributeur selon la revendication 1, dans lequel la réserve de produit volatil (78) est suspendue dans un porte-réserve (82).

5. Distributeur selon la revendication 4, dans lequel le porte-réserve (82) comprend un dispositif de protection de bord résistant à la chaleur (83) protégeant son bord inférieur contre une surchauffe indésirable.

6. Distributeur selon la revendication 2, dans lequel ladite réserve de produit volatil (78, 144) a, au niveau de son bord supérieur, une partie (93) de coupe transversale gênante par rapport à ladite fente, moyennant quoi la réserve est suspendue dans la chambre de chauffage par ladite partie de coupe transversale gênante (93).

7. Distributeur de produits volatils chauffés selon la revendication 6, dans lequel la fente d'insertion comprend des structures de calage qui donnent un profil de coupe transversale à la fente d'insertion qui restreint l'accès à celle-ci de manière telle à empêcher l'insertion à travers la fente d'une réserve quelconque de produit volatil (88, 89) non capable de présenter un profil de coupe transversale non gênant vis-à-vis du profil de coupe transversale de la fente d'insertion.

8. Distributeur de produits volatils chauffés selon la revendication 6, dans lequel les structures de calage définissent un profil de coupe transversale choisi parmi le groupe constitué de parties courbées et se croisant de façon angulaire.

9. Distributeur de produits volatils chauffés selon l'une quelconque des revendications précédentes, dans lequel le brûleur de combustible (118) supporte une flamme positionnée à l'intérieur de la chambre de chauffage et les parois de la chambre de chauffage comprennent une partie de transmission de lumière (114) qui permet à la lumière provenant de la flamme d'être visible à un utilisateur du distributeur à travers la partie de transmission de lumière (114).

10. Distributeur de produits volatils chauffés selon la revendication 9, dans lequel
a. les parois d'une partie de la chambre de chauffage sont choisies parmi le groupe constitué de matériaux opaques et translucides afin d'être obstruée visuellement, et
b. l'emplacement au niveau duquel une réserve de produit volatil est maintenue à l'intérieur de la chambre de chauffage est à l'intérieur de la partie obstruée visuellement de la chambre de chauffage.

11. Distributeur de produits volatils chauffés selon l'une quelconque des revendications précédentes, dans lequel le brûleur de combustible (118) brûle un combustible choisi parmi le groupe constitué d'une bougie, un solide brûlable, un gaz sous pression, un liquide combustible, et un liquide combustible sous forme de gel.

12. Distributeur de produits volatils chauffés selon la revendication 1, dans lequel le brûleur de combustible (118) est ventilé à partir d'en dessous par l'intermédiaire d'un espace ouvert s'étendant de façon circonférentielle (129) entourant le brûleur de combustible (118), lequel espace est ventilé vers l'air ambiant.

13. Distributeur de produits volatils chauffés selon l'une quelconque des revendications précédentes, comprenant une base (112) située en dessous de la chambre de chauffage (114), la base (112) ayant une ouverture de ventilation (136, 129) à travers laquelle de l'air ambiant peut passer pour continuer vers le haut à travers la chambre de chauffage (114) et dans lequel le brûleur de combustible (118) est supporté par la base par rapport à l'ouverture de ventilation de manière telle que le brûleur de combustible est ventilé de façon circonférentielle à partir d'en dessous.

14. Distributeur de produits volatils chauffés selon l'une quelconque des revendications précédentes, dans lequel le brûleur de combustible utilise une bougie.

15. Distributeur de produits volatils chauffés selon la revendication 13, dans lequel le brûleur de combustible (118) comprend une coupelle à bougie (116) ayant des parois de bougie et une douille s'ouvrant vers le bas, et la base comprend un montant de fixation pour venir en prise avec la douille et ainsi maintenir la coupelle à bougie (116).

16. Distributeur de produits volatils chauffés selon la revendication 13, dans lequel la chambre de chauffage comprend une cheminé de transmission de lumière attachée à la base.

17. Distributeur de produits volatils chauffés selon la revendication 13, comprenant un dispositif de suspension (156) par lequel le distributeur peut être suspendu à partir du dessus.

18. Distributeur de produits volatils chauffés selon la revendication 17, dans lequel le dessous de la base (112a) est formé de manière telle à empêcher le distributeur d'être supporté dans une orientation verticale sur une surface plate.

19. Distributeur de produits volatils chauffés selon la revendication 1, dans lequel
a. le brûleur de combustible (118) comprend une bougie contenue à l'intérieur d'une coupelle à bougie (116), la coupelle à bougie (116) ayant un fond (122) et des parois verticales (120) se terminant dans une partie supérieure ouverte et étant faits d'un matériau résistant à la chaleur, et
b. la coupelle à bougie (116) est positionnée de façon fixe à l'intérieur de la chambre de chauffage (114), de telle sorte que, si le distributeur de produits volatils penche vers le côté alors que la bougie est en train de brûler, la chaleur de la bougie contenue à l'intérieur de la coupelle à bougie fera fondre au moins une partie de la cire de la bougie, lui permettant de couler à partir de la partie supérieure ouverte pour priver la mèche de la bougie de combustible, forçant la bougie à s'auto-éteindre.

20. Distributeur de produits volatils selon la revendication 3, dans lequel le dispositif de protection de bord a des aubes de déflection s'étendant vers le côté par rapport à la direction de surface de la réserve de produit volatil (78).

21. Distributeur de produits volatils chauffés selon la revendication 1, dans lequel le brûleur de combustible est choisi parmi le groupe constitué d'une bougie, un liquide combustible solidifié, un solide brûlable, un dispositif de chauffage catalytique, un brûleur de gaz sous pression, et une mèche qui est alimentée avec un liquide combustible.

22. Distributeur selon l'une quelconque des revendications précédentes, dans lequel ladite réserve de produit volatil (144) porte une quantité choisie du produit volatil devant être distribué et il est proposé une source de combustible pour le brûleur de combustible (118), la quantité de combustible dans la source de combustible étant choisie pour être épuisée sensiblement en même temps qu'une quantité choisie du produit volatil a été épuisée à partir de la réserve de produit volatil (144), moyennant quoi l'épuisement de combustible propose un signal d'utilisation complète indiquant que la quantité choisie de produit volatil a également été épuisée.

23. Procédé pour distribuer des ingrédients volatilisables par application de chauffage, le procédé comprenant les étapes consistant à :
a. proposer un distributeur de produits volatils chauffés (110) ayant :
i. une réserve de produit volatil (144) avec des surfaces majeures exposées, une avant et une arrière, pour libérer un matériau volatil ;
ii. une chambre de chauffage fermée (114) ayant des parois de chambre et étant ventilée vers l'air extérieur ;
iii. un brûleur de combustible (118) ; et
iv. un passage d'écoulement d'air (114, 140) pour guider les gaz chauds à partir du brûleur de combustible (118) au-delà d'une réserve de produit volatil maintenue à l'intérieur de la chambre de chauffage pour chauffer la réserve de produit volatil par l'intermédiaire de l'exposition directe de la réserve de produit volatil (144) aux gaz chauds, le passage d'écoulement d'air dirigeant alors les gaz chauds pour être évacués à partir du distributeur vers l'air extérieur ;
b. positionner la réserve de produit volatil (144) chargée avec des ingrédients volatils dans la chambre de chauffage (114) dans une orientation telle que les gaz chauds passent le long de la surface majeure exposée dans une direction généralement verticale généralement parallèle à la direction de prolongement linéaire des surfaces exposées de la réserve de produit volatil (144), l'orientation exposant à la fois les côtés avant et arrière de la réserve de produit volatil (144) à l'écoulement de gaz chauds ;
c. allumer le combustible au niveau du brûleur (118) ; et
d. permettre aux ingrédients ainsi volatilisés à partir de la réserve de produit volatil (144) d'être évacués à partir du distributeur.

24. Procédé selon la revendication 23, comprenant en outre l'étape de proposer une source de combustible pour le brûleur de combustible (118) ayant une quantité de combustible choisie pour s'épuiser, forçant le brûleur de combustible à s'éteindre, en même temps que les ingrédients volatils sur la réserve de produit volatil sont sensiblement épuisés de telle sorte que l'extinction du brûleur de combustible est un signal d'utilisation complète pour l'épuisement sensible des ingrédients volatils à partir de la réserve de produit volatil.
